# EUROPÄISCHE PATENTSCHRIFT

(11) **EP 1 633 346 B1**
(45) Veröffentlichungstag und Bekanntmachung des Hinweises auf die Patenterteilung: **23.08.2006**
(21) Anmeldenummer: 04735007.9
(22) Anmeldetag: 27.05.2004
(51) Int. Cl.: A61K 31/4025, C07D 401/12, C07D 413/12, C07D 403/12, C07C 275/26, A61P 7/02

(54) **PYRROLIDIN-1,2-DICARBONSÄURE-1-¬(4-ETHINYL-PHENYL)-AMID|-2-¬(PHENYL)-AMID| DERIVATE ALS INHIBITOREN DER KOAGULATIONSFAKTOREN XA UND VIIA ZUR BEHANDLUNG VON THROMBOSEN**
PYRROLIDIN-1, 2- DICARBOXYLIC ACID- 1- (4- ETHINYL- PHENYL) - AMID|- 2- (PHENYL)- AMID| DERIVATIVE AS INHIBITORS OF COAGULATION FACTOR XA AND VIIA FOR THE TREATMENT OF THROMBOSIS
DERIVES D'ACIDE PYRROLIDINE-1,2-DICARBOXYLIQUE-1- (4-ETHINYL-PHENYL)-AMIDE|-2- (PHENYL)-AMIDE| UTILISES COMME INHIBITEURS DES FACTEURS DE COAGULATION XA ET VIIA POUR LE TRAITEMENT DE THROMBOSES

(30) Priorität: 18.06.2003 DE 10327428; 01.07.2003 DE 10329457
(43) Veröffentlichungstag der Anmeldung: 15.03.2006
(73) Patentinhaber: Merck Patent GmbH, 64293 Darmstadt (DE)
(72) Erfinder: MEDERSKI, Werner, 64673 Zwingenberg (DE); TSAKLAKIDIS, Christos, 69469 Weinheim (DE); DORSCH, Dieter, 64372 Ober-Ramstadt (DE); CEZANNE, Bertram, 64546 Mörfelden-Walldorf (DE); GLEITZ, Johannes, 64293 Darmstadt (DE)
(86) Internationale Anmeldenummer: PCT/EP2004/005717
(87) Internationale Veröffentlichungsnummer: WO 2004/110433

(56) Entgegenhaltungen:
- WO-A-02/079145
- WO-A-03/045912

## Beschreibung

Die Erfindung betrifft Verbindungen der Formel 1 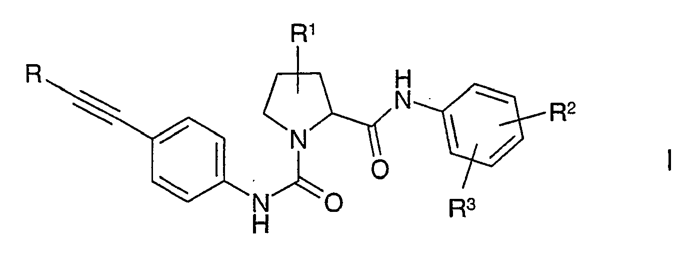 worin
- R: H, X, A, X-CO- oder A-CO-,
- R¹: H, =O, Hal, X, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)m-O-, -O(CH₂)ₘCOOH oder -O(CH₂)ₘOA,
- R²: H, Hal oder A,
- R³: einen einkernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, CN, (CH₂)ₙOH, NR⁴R⁵, =NH, =N-OH, =N-OA, COOA und/oder Carbonylsauerstoff (=O) substituiert sein kann, oder CONR⁴R⁵,
- R² und R³: zusammen auch -CH=CH-NH- oder -CH₂-CH₂-NH, wobei ein H-Atom durch A-CO- oder A-O-CO- ersetzt sein kann,
- R⁴, R⁵: unabhängig voneinander H oder A,
- R⁴ und R⁵: zusammen auch eine Alkylenkette mit 3, 4 oder 5 C-Atomen, die auch durch A, Hal, OA und/oder Carbonylsauerstoff (=CO) substituiert sein kann,
- X: Aryl, Arylalkyl, Het oder Het-alkyl,
- Aryl: unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, Benzyl, Cycloalkyl, OH, NH₂, NHCONH₂, NO₂, CN, -CH₂-COOH, -CH₂-CONH₂, NHCOA, NR³SO₂A, CHO, SO₂NH₂, SO₂A und/oder Carbonylsauerstoff substituiert sein kann,
- A: unverzweigtes, verzweigtes oder cylisches Alkyl mit 1-10 C-Atomen, worin auch 1-7 H-Atome durch F und/oder Chlor ersetzt sein können,
- Hal: F, Cl, Br oder I,
- m: 1, 2, 3, 4, 5 oder 6,
- n: 0, 1, 2, 3, 4 , 5 oder 6
bedeuten,
sowie ihre pharmazeutisch verwendbaren Salze, Solvate und Stereoisomere, einschließlich deren Mischungen in allen Verhältnissen.

Gegenstand der Erfindung sind auch die optisch aktiven Formen, die Racemate, die Diastereomeren sowie die Hydrate und Solvate, z.B. Alkoholate, dieser Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie Faktor Xa inhibierende Eigenschaften und können daher zur Bekämpfung und Verhütung von thromboembolischen Erkrankungen wie Thrombose, myocardialem Infarkt, Arteriosklerose, Entzündungen, Apoplexie, Angina pectoris, Restenose nach Angioplastie und Claudicatio intermittens eingesetzt werden.

Die erfindungsgemäßen Verbindungen der Formel I sind weiterhin Inhibitoren der Gerinnungsfaktoren Faktor VIIa, Faktor IXa und Thrombin der Blutgerinnungskaskade sein.

Andere Ethinylderivate sind als Faktor Xa-Inhibitoren in der WO 02/079145 beschrieben.
Andere aromatische Amide sind in der WO 99/00121 und in der WO 00/39118 beschrieben. Aromatische Amidinderivate mit antithrombotischer Wirkung sind z.B. aus der EP 0 540 051 B1 bekannt. Cyclische Guanidine zur Behandlung thromboembolischer Erkrankungen sind z.B. in der WO 97/08165 beschrieben. Aromatische Heterocyclen mit Faktor Xa inhibitorischer Aktivität sind z.B. aus der WO 96/10022 bekannt. Substituierte N-[(Aminoiminomethyl)phenylalkyl]-azaheterocyclylamide als Faktor Xa Inhibitoren sind in WO 96/40679 beschrieben.

Der antithrombotische und antikoagulierende Effekt der erfindungsgemäßen Verbindungen wird auf die inhibierende Wirkung gegenüber der aktivierten Gerinnungsprotease, bekannt unter dem Namen Faktor Xa, oder auf die Hemmung anderer aktivierter Serinproteasen wie Faktor VIIa, Faktor IXa oder Thrombin zurückgeführt.

Faktor Xa ist eine der Proteasen, die in den komplexen Vorgang der Blutgerinnung involviert ist. Faktor Xa katalysiert die Umwandlung von Prothrombin in Thrombin. Thrombin spaltet Fibrinogen in Fibrinmonomere, die nach Quervernetzung elementar zur Thrombusbildung beitragen. Eine Aktivierung von Thrombin kann zum Auftreten von thromboembolischen Erkrankungen führen. Eine Hemmung von Thrombin kann jedoch die in die Thrombusbildung involvierte Fibrinbildung inhibieren.

Die Messung der Inhibierung von Thrombin kann z.B. nach der Methode von G. F. Cousins et al. in *Circulation* **1996**, 94, 1705-1712 erfolgen.

Eine Inhibierung des Faktors Xa kann somit verhindern, daß Thrombin gebildet wird.
Die erfindungsgemäßen Verbindungen der Formel I sowie ihre Salze greifen durch Inhibierung des Faktors Xa in den Blutgerinnungsprozeß ein und hemmen so die Entstehung von Thromben.

Die Inhibierung des Faktors Xa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Hauptmann et al. in *Thrombosis and Haemostasis* **1990,** *63,* 220-223 beschrieben.

Die Messung der Inhibierung von Faktor Xa kann z.B. nach der Methode von T. Hara et al. in *Thromb. Haemostas.* **1994**, *71*, 314-319 erfolgen.

Der Gerinnungsfaktor Vlla initiiert nach Bindung an Tissue Faktor den extrinsischen Teil der Gerinnungskaskade und trägt zur Aktivierung des Faktors X zu Faktor Xa bei. Eine Inhibierung von Faktor VIIa verhindert somit die Entstehung des Faktors Xa und damit eine nachfolgende Thrombinbildung.
Die Inhibierung des Faktors VIIa durch die erfindungsgemäßen Verbindungen und die Messung der antikoagulierenden und antithrombotischen Aktivität kann nach üblichen in vitro- oder in vivo-Methoden ermittelt werden. Ein übliches Verfahren zur Messung der Inhibierung von Faktor VIIa wird z.B. von H. F. Ronning et al. in *Thrombosis Research* **1996***, 84,* 73-81 beschrieben.

Der Gerinnungsfaktor IXa wird in der intrinsischen Gerinnungskaskade generiert und ist ebenfalls an der Aktivierung von Faktor X zu Faktor Xa beteiligt. Eine Inhibierung von Faktor IXa kann daher auf andere Weise verhindern, daß Faktor Xa gebildet wird.
Die Inhibierung von Faktor IXa durch die erfindungsgemäßen Verbindungen und die Messung der antikoagulierenden und antithrombotischen Aktivität kann nach üblichen in vitro- oder in vivo-Methoden ermittelt werden. Ein geeignetes Verfahren wird z.B. von J. Chang et al. in *Journal of Biological Chemistry* **1998,** *273*, 12089-12094 beschrieben.

Die erfindungsgemäßen Verbindungen können weiterhin zur Behandlung von Tumoren, Tumorerkrankungen und/oder Tumormetastasen verwendet werden.
Ein Zusammenhang zwischen dem Tissuefaktor TF / Faktor VIIa und der Entwicklung verschiedener Krebsarten wurde von T.Taniguchi und N.R.Lemoine in Biomed. Health Res. (2000), 41 (Molecular Pathogenesis of Pancreatic Cancer), 57-59, aufgezeigt. Die im nachfolgenden aufgeführten Publikationen beschreiben eine antitumorale Wirkung von TF-VII und Faktor Xa Inhibitoren bei verschiedenen Tumorarten:
K.M. Donnelly et al. in Thromb. Haemost. 1998; 79: 1041-1047;
E.G. Fischer et al. in J. Clin. Invest. 104: 1213-1221 (1999);
B.M. Mueller et al. in J. Clin. Invest. 101: 1372-1378 (1998);
M.E. Bromberg et al. in Thromb. Haemost. 1999; 82: 88-92

Die Verbindungen der Formel I können als Arzneimittelwirkstoffe in der Human- und Veterinärmedizin eingesetzt werden, insbesondere zur Behandlung und Verhütung von thromboembolischen Erkrankungen wie Thrombose, myocardialem Infarkt, Arteriosklerose, Entzündungen, Apoplexie, Angina pectoris, Restenose nach Angioplastie, Claudicatio intermittens, venöse Thrombose, pulmonale Embolie, arterielle Thrombose, myocardiale Ischämie, instabile Angina und auf Thrombose basierender Schlaganfall.
Die erfindungsgemäßen Verbindungen werden auch zur Behandlung oder Prophylaxe von atherosklerotischen Erkrankungen wie koronarer arterieller Erkrankung, cerebraler arterieller Erkrankung oder peripherer arterieller Erkrankung eingesetzt.
Die Verbindungen werden auch in Kombination mit anderen Thrombolytika bei myocardialem Infarkt eingesetzt, ferner zur Prophylaxe zur Reocclusion nach Thrombolyse, percutaner transluminaler Angioplastie (PTCA) und koronaren Bypass-Operationen.
Die erfindungsgemäßen Verbindungen werden ferner verwendet zur Prävention von Rethrombose in der Mikrochirurgie, ferner als Antikoagulantien im Zusammenhang mit künstlichen Organen oder in der Hämodialyse.
Die Verbindungen finden ferner Verwendung bei der Reinigung von Kathetern und medizinischen Hilfsmitteln bei Patienten *in vivo,* oder als Antikoagulantien zur Konservierung von Blut, Plasma und anderen Blutprodukten *in vitro.* Die erfindungsgemäßen Verbindungen finden weiterhin Verwendung bei solchen Erkrankungen, bei denen die Blutkoagulation entscheidend zum Erkrankungsverlauf beiträgt oder eine Quelle der sekundären Pathologie darstellt, wie z.B. bei Krebs einschließlich Metastasis, entzündlichen Erkrankungen einschließlich Arthritis, sowie Diabetes.

Die erfindungsgemäßen Verbindungen finden weiterhin Verwendung zur Behandlung von Migräne (F. Morales-Asin et al., Headache, 40, 2000, 45-47). Darüberhinaus können sie zur Behandlung von Tinnitus verwendet werden. Die Verwendung von Antikoagulantien bei der Tinnitustherapie ist von R. Mora et al. in International Tinnitus Journal (2003), 9(2), 109-111 beschrieben.

Bei der Behandlung der beschriebenen Erkrankungen werden die erfindungsgemäßen Verbindungen auch in Kombination mit anderen thrombolytisch wirksamen Verbindungen eingesetzt, wie z.B. mit dem "tissue plasminogen activator" t-PA, modifiziertem t-PA, Streptokinase oder Urokinase. Die erfindungsgemäßen Verbindungen werden mit den anderen genannten Substanzen entweder gleichzeitig oder vorher oder nachher gegeben.
Besonders bevorzugt ist die gleichzeitige Gabe mit Aspirin, um ein Neuauftreten der Thrombenbildung zu verhindern.
Die erfindungsgemäßen Verbindungen werden auch verwendet in Kombination mit Blutplättchen-Glycoprotein-Rezeptor (IIb/IIIa)-Antagonisten, die die Blutplättchenaggregation inhibieren.

Gegenstand der Erfindung sind die Verbindungen der Formel I und ihre Salze sowie ein Verfahren zur Herstellung von Verbindungen der Formel 1 nach Anspruch 1 sowie ihrer Salze, dadurch gekennzeichnet, daß man
a) eine Verbindung der Formel II 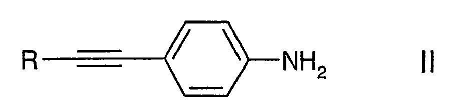 worin R die in Anspruch 1 angegebene Bedeutung hat,
   mit einem Chloroformiatderivat zu einem intermediären Carbamatderivat umsetzt,
   das anschließend mit einer Verbindung der Formel III 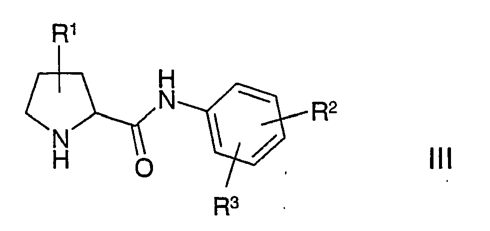 worin
   R¹, R² und R³ die in Anspruch 1 angegebene Bedeutung haben,
   umgesetzt wird,
   oder
b) eine Verbindung der Formel III
   mit einer Verbindung der Formel IV 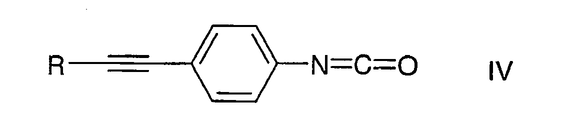 worin
   R die in Anspruch 1 angegebene Bedeutung hat,
   umsetzt,
   oder
c) eine Verbindung der Formel V 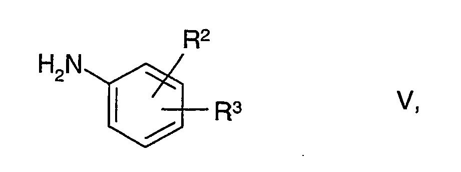 worin R² und R³ die in Anspruch 1 angegebene Bedeutung haben,
   mit einer Verbindung der Formel VI 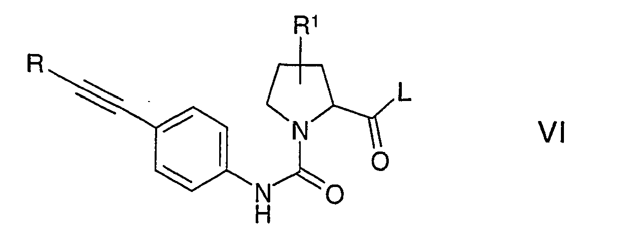
worin
- L: Cl, Br, I oder eine freie oder reaktionsfähig funktionell abgewandelte OH-Gruppe bedeutet und
- R und R¹: die in Anspruch 1 angegebenen Bedeutungen haben,
umsetzt,
und/oder eine Base oder Säure der Formel I in eines ihrer Salze umwandelt.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, gilt, daß deren Bedeutungen unabhängig voneinander sind.

Vor- und nachstehend haben die Reste bzw. Parameter R, R¹, R² und R³, die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl.
A bedeutet auch Cycloalkyl. Cycloalkyl hat vorzugsweise 3-7 C-Atome bedeutet vorzugsweise

Hal bedeutet vorzugsweise F, Cl oder Br, aber auch 1.

R¹ bedeutet vorzugsweise H, =O, Hal, Aryl, Het, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)ₘ-O-, -O(CH₂)ₘCOOH oder -O(CH₂)ₘOA, besonders bevorzugt ist H, OH, O-Allyl, O-Propargyl, OCH₂CH(OH)CH₂OH, A-O-CO-(CH₂)ₘ-O-, wie z.B. Methoxycarbonylmethoxy; -O(CH₂)ₘCOOH, wie z.B. Carboxymethoxy; OA, wie z.B wie z.B. Methoxy oder Ethoxy; oder O(CH₂)ₘOA, wie z.B. Methoxyethoxy.

R³ bedeutet vorzugsweise einen einkernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, =NH, OH, COOA und/oder Carbonylsauerstoff (=O) substituiert sein kann, oder R³ bedeutet CONR⁴R⁵.

Insbesondere bedeutet R³ 2-Oxo-piperidin-1-yl, 2-Oxo-pyrrolidin-1-yl, 2-Oxo-1 *H*-pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1 *H*-Pyrazin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl, 4*H*-[1,4]Oxazin-4-yl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, gegebenenfalls ein- oder zweifach durch Hal, OH, COOA, OA und/oder A substituiert,
oder
CONR⁴R⁵.

R³ bedeutet besonders bevorzugt 2-Oxo-piperidin-1-yl, 2-Oxo-pyrrolidin-1-yl, 2-Oxo-1*H*-pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl, 3-Oxo-2*H*-pyridazin-2-yl,
Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OH, COOA, OA und/oder A substituiert,
oder CONR⁴R⁵, wobei
R⁴ und R⁵ zusammen eine Alkylenkette mit 3, 4 oder 5 C-Atomen bedeutet.

R² und R³ bedeuten zusammen auch -CH=CH-NH- oder -CH₂-CH₂-NH, wobei ein H-Atom durch A-CO- oder A-O-CO- ersetzt sein kann, wie z.B. durch Acetyl oder Ethoxycarbonyl.

Aryl bedeutet vorzugsweise unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂,SO₂A, -CH₂-COOH oder -OCH₂-COOH substituiertes Phenyl, Naphthyl oder Biphenyl.
Aryl bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m-oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methylsulfonamido)-phenyl, o-, m- oder p-(Methylsulfonyl)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

"Alkyl" in Arylalkyl oder Het-alkyl bedeutet z.B. Methylen, Ethylen oder Propylen.

Het bedeutet z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6-oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2, 1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4-oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ij ausgedrückt werden, die der Formel I entsprechen und worin die nicht näher bezeichneten Reste die bei der Formel I angegebene Bedeutung haben, worin jedoch
in la
- R: H oder A bedeutet;
in Ib
- R³: einen einkernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, =NH, OH, COOA und/oder Carbonylsauerstoff (=O) substituiert sein kann,
oder CONR⁴R⁵
bedeutet;
in Ic
- R³: 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H-*Pyrazin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl, 4*H*-[1,4]Oxazin-4-yl,
Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OA, OH, COOA und/oder A substituiert,
oder
CONR⁴R⁵,
- R⁴, R⁵: unabhängig voneinander H oder A,
- R⁴ und R⁵: zusammen auch eine Alkylenkette mit 3, 4 oder 5 C-Atomen,
bedeuten;
in Id
- R: H, X, A, X-CO- oder A-CO-,
- R¹: H, =O, Hal, X, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)ₘ-O-, -O(CH₂)ₘCOOH oder -O(CH₂)ₘOA,
- R²: H, Hal oder A,
- R³: 2-Oxo-piperidin-1-yl, 2-Oxo-pyrrolidin-1-yl, 2-Oxo-1*H* pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OA, OH, COOA und/oder A substituiert,
oder
CONR⁴R⁵,
- R⁴, R⁵: unabhängig voneinander H oder A,
- R⁴ und R⁵: zusammen auch eine Alkylenkette mit 3, 4 oder 5 C-Atomen,
- X: Aryl, Arylalkyl, Het oder Het-alkyl,
- Aryl: unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der unsubstituiert oder ein-, zwei- oder dreifach durch Hal, A, Benzyl, Cycloalkyl, OH, NH₂, NHCONH₂, NO₂, CN, -CH₂-COOH, -CH₂-CONH₂, NHCOA, NR³SO₂A, CHO, SO₂NH₂, SO₂A und/oder Carbonylsauerstoff substituiert sein kann,
- A: unverzweigtes, verzweigtes oder cylisches Alkyl mit 1-10 C-Atomen, worin auch 1-7 H-Atome durch F ersetzt sein können,
- Hal: F, Cl, Br oder I,
bedeuten;
in le
- R: H oder A,
- R¹: H, OH, OA, O-Allyl, O-Propargyl, OCH₂CH(OH)CH₂OH, A-O-CO-(CH₂)ₘ-O-, -O(CH₂)ₘCOOH oder -O(CH₂)ₘOA,
- R²: H, Hal oder A,
- R³: 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl, 3-Oxo-2*H*-pyridazin-2-yl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OA, OH, COOA und/oder A substituiert,
oder CONR⁴R⁵,
- R⁴ und R⁵: zusammen eine Alkylenkette mit 3, 4 oder 5 C-Atomen,
- A: unverzweigtes, verzweigtes oder cylisches Alkyl mit 1-10 C-Atomen, worin auch 1-7 H-Atome durch F ersetzt sein können,
- Hal: F, Cl, Br oder I,
bedeuten;
in If
- R: H, X, A, X-CO- oder A-CO-,
- R¹: H, =O, Hal, X, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)ₘ-O-, -O(CH₂)ₘCOOH oder -O(CH₂)ₘOA,
- R²: H, Hal oder A,
- R³: 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
- X: Aryl, Arylalkyl, Het oder Het-alkyl,
- Aryl: unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
- Het: einen ein- oder zweikernigen gesättigten, ungesättigten oder aromatischen Heterocyclus mit 1 bis 4 N-, O-und/oder S-Atomen, der unsubstituiert oder ein-, zwei- oder dreifach durch Hal, A, Benzyl, Cycloalkyl, OH, NH₂, NHCONH₂, NO₂, CN, -CH₂-COOH, -CH₂-CONH₂, NHCOA, NR³SO₂A, CHO, SO₂NH₂, SO₂A und/oder Carbonylsauerstoff substituiert sein kann,
- A: unverzweigtes, verzweigtes oder cylisches Alkyl mit 1-10 C-Atomen, worin auch 1-7 H-Atome durch F ersetzt sein können,
- Hal: F, Cl, Br oder I,
bedeuten;
in Ig
- R³: 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl,
bedeutet;
in Ih
- R¹: H, OH, OA, O-Allyl, O-Propargyl, OCH₂CH(OH)CH₂OH, A-O-CO-(CH₂)ₘ-O-, -O(CH₂)ₘCOOH oder -O(CH₂)ₘOA,
bedeutet,
in li
- A: unverzweigtes oder verzweigtes Alkyl mit 1-6 C-Atomen
bedeutet;
in Ij
- R: H oder A,
- R¹: H, OH, OA, O-Allyl, O-Propargyl, OCH₂CH(OH)CH₂OH, A-O-CO-(CH₂)ₘ-O-, -O(CH₂)ₘCOOH oder -O(CH₂)ₘOA,
- R²: H, Hal oder A,
- R³: 2-Oxo-piperidin-1-yl, 2-Oxo-pyrrolidin-1-yl, 2-Oxo-1*H-*pyridin-1-yl, 3-Oxo-Morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl, gegebenenfalls einfach durch A, OH, COOA substituiert,
- A: unverzweigtes, verzweigtes oder cylisches Alkyl mit 1-10 C-Atomen, worin auch 1-7 H-Atome durch F ersetzt sein können,
- Hal: F, Cl, Br oder I,
sowie ihre pharmazeutisch verwendbaren Salze, Solvate und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise erhalten werden, indem man Verbindungen der Formel II mit einem Chloroformiatderivat, z.B. 4-Nitrophenylchlorformiat zu einem intermediären Carbamat umsetzt und anschließend mit Verbindungen der Formel III umsetzt.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.

Als inerte Lösungsmittel eignen sich z.B. Wasser; Kohlenwasserstoffe wie Hexan, Petrolether, Benzol, Toluol oder Xylol; chlorierte Kohlenwasserstoffe wie Trichlorethylen, 1 ,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Ausgangsverbindungen der Formeln II und III sind in der Regel bekannt. Sind sie neu, so können sie aber nach an sich bekannten Methoden hergestellt werden.

Verbindungen der Formel I können auch erhalten werden, indem man Verbindungen der Formel III mit Verbindungen der Formel IV umsetzt.

Dies erfolgt unter Bedingungen wie oben beschrieben.
Die Ausgangsverbindungen der Formel IV sind in der Regel bekannt. Sind sie neu, so können sie aber nach an sich bekannten Methoden hergestellt werden.

Verbindungen der Formel I können auch erhalten werden, indem man Verbindungen der Formel V mit Verbindungen der Formel VI umsetzt.
In den Verbindungen der Formel VI bedeutet L vorzugsweise Cl, Br, I oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, in Gegenwart eines säurebindenden Mittels vorzugsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums. Auch der Zusatz einer organischen Base wie Triethylamin, Dimethylanilin, Pyridin oder Chinolin oder eines Überschusses der Aminkomponente der Formel IV kann günstig sein. Die Reaktionszeit liegt je nach den angewendeten Bedingungen zwischen einigen Minuten und 14 Tagen, die Reaktionstemperatur zwischen etwa 0° und 150°, normalerweise zwischen 20° und 130°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Verbindungen der Formel VI sind in der Regel neu und werden durch Umsetzung der Verbindungen der Formel IV mit Verbindungen der Formel VII 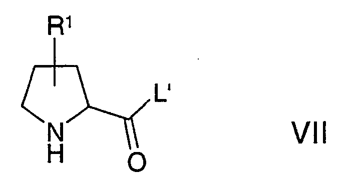 erhalten. In den Verbindungen der Formel VII bedeutet L' z.B. OH und R¹ hat die in Anspruch 1 angegebene Bedeutung.

Ester können z.B. mit Essigsäure oder mit NaOH oder KOH in Wasser, Wasser-THF oder Wasser-Dioxan bei Temperaturen zwischen 0 und 100° verseift werden.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden.
Auch physiologisch unbedenkliche organische Basen, wie z.B. Ethanolamin können verwendet werden.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereo-isomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktive Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I und/oder ihre pharmazeutisch verwendbaren Solvate und Stereoisomere, einschließlich deren Mischungen in allen Verhältnissen, sowie gegebenenfalls Träger- und/oder Hilfsstoffe.

Diese Arzneimittel können in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder oder auch als Nasenspray. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung und Verhütung von thrombo-embolischen Erkrankungen wie Thrombose, myocardialem Infarkt, Arteriosklerose, Entzündungen, Apoplexie, Angina pectoris, Restenose nach Angioplastie, Claudicatio intermittens, Migräne, Tumoren, Tumorerkrankungen und/oder Tumormetastasen verwendet werden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

Gegenstand der Erfindung ist ferner die Verwendung von Verbindungen der Formel I und/oder ihrer pharmazeutisch verwendbaren Salze, Solvate und Stereoisomere, einschließlich deren Mischungen in allen Verhältnissen,
zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tinnitus, Tumoren, Tumorerkrankungen und/oder Tumormetastasen, in Kombination mit mindestens einem weiteren Arzneimittelwirkstoff.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
Massenspektrometrie (MS): El (Elektronenstoß-lonisation) M⁺
ESI (Electrospray lonization) (M+H)⁺
FAB (Fast Atom Bombardment) (M+H)⁺

### Beispiel 1

Die Herstellung von (2R,4R)-4-Methoxy-pyrrolidin-1,2-dicarbonsäure-1-[(4-ethinyl-phenyl)-amid]-2-{[4-(2-oxo-2*H*-pyridin-1-yl)-phenyl]-amid} ("A1") erfolgt analog nachstehendem Schema: 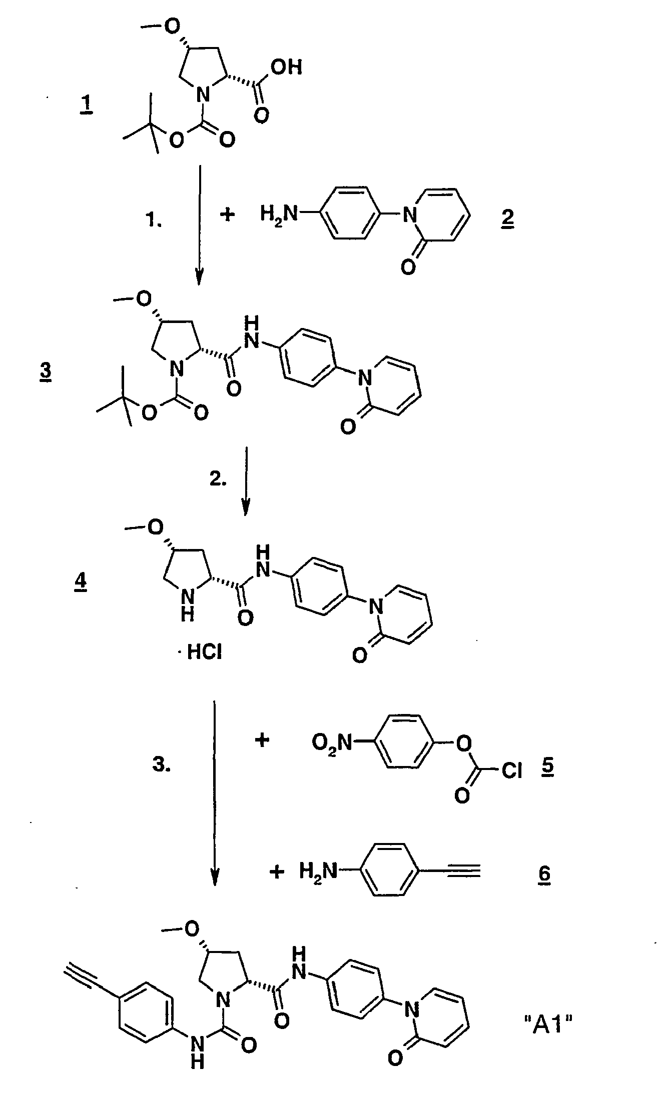
1. 1,0 g (4,076 mmol) BOC-methoxy-prolin **1** als Suspension in 15 mL Toluol werden bei Raumtemperatur nacheinander unter Rühren mit 0,76 g (4,076 mmol) Anilino-pyridon **2** und 1,008 g (4,076 mmol) Ethyl-2-ethoxy-1,2-dihydrochinolin-1-carboxylat versetzt. Anschliessend wird bei dieser Temperatur 18 h gerührt. Danach wird wie üblich aufgearbeitet und man erhält so 1,53 g (90,8%) BOC-prolin-anilid-derivat 3 als Rohprodukt; MS-EI (M⁺) 414.
2. 1,5 g (3,628 mmol) **3** werden in 20 mL Dioxan gelöst und bei Raumtemperatur mit 20 mL 4 N HCl in Doxan versetzt und 2 h bei dieser Temperatur gerührt. Danach wird wie üblich aufgearbeitet und man erhält so 0,94 g (74,1%) das Prolin-anilid-derivat-Hydrochlorid **4** als Rohprodukt.
3. 202 mg (1,001 mmol) 4-Nitrophenylchlorformiat **5**, 118mg (1,001 mmol) 4-Ethinylanilin und 0,081 mL (1,001 mmol) Pyridin in 10 mL Dichlormethan werden unter Stickstoffatmosphäre 1 h bei Raumtemperatur gerührt. Anschliessend werden 350 mg (1,001 mmol) **4** und 0,511 mL (3,003 mmol) N-Ethyldiisopropylamin in 5mL Dichlormethan zugegeben. Die entstandene Suspension wird noch 2 h bei Raumtemperatur gerührt. Danach wird wie üblich aufgearbeitet und man erhält so 185 mg (40,5%) "A1"; MS-El (M⁺) 457.

### Analog erhält man die nachstehenden Verbindungen

(2R,4R)-4-Methoxy-pyrrolidin-1,2-dicarbonsäure-1-[(4-ethinyl-phenyl)-amid]-2-{[4-(3-oxo-morpholin-4-yl)-phenyl]-amid} ("A2"),
(2R,4R)-4-Methoxy-pyrrolidin-1,2-dicarbonsäure-1-[(4-ethinyl-phenyl)-amid]-2-{[3-methyl-4-(3-oxo-morpholin-4-yl)-phenyl]-amid} ("A3"),
(2R,4R)-4-Hydroxy-pyrrolidin-1,2-dicarbonsäure-1-[(4-ethinyl-phenyl)-amid]-2-{[4-(3-oxo-morpholin-4-yl)-phenyl]-amid},
(2R,4R)-4-Ethoxy-pyrrotidin-1,2-dicarbonsäure-1-[(4-ethinyl-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Hydroxy-pyrrolidin-1,2-dicarbonsäure-1-[(4-ethinyl-phenyl)-amid]-2-{[3-methyl-4-(3-oxo-morpholin-4-yl)-phenyl]-amid},
(2R)-Pyrrolidin-1,2-dicarbonsäure-1-[(4-ethinyl-phenyl)-amid]-2-{[4-(3-oxo-morpholin-4-yl)-phenyl]-amid},
(2R)-Pyrrolidin-1,2-dicarbonsäure-1-[(4-ethinyl-phenyl)-amid]-2-{[3-methyl-4-(3-oxo-morpholin-4-yl)-phenyl]-amid},
(2R)-Pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[2-fluor-4-(3-oxo-morpholin-4-yl)-phenyl]-amid}, F. 191-192°;
(2R,4R)-4-Ethoxy-pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Ethoxy-pyrrolidin-1,2-dicarbonsäure-1-[(4-ethinyl-phenyl)-amid]-2-{[3-methyl-4-(3-oxo-morpholin-4-yl)-phenyl]-amid},
(2R)-Pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[4-(2-oxo-1*H*-pyrazin-1-yl)-phenyl]-amid}.

### Beispiel 2

### Analog Beispiel 1 erhält man die nachstehenden Verbindungen

(2R,4R)-4-Hydroxy-pyrrolidin-1,2-dicarbonsäure-1-[(4-ethinyl-phenyl)-amid]-2-{[4-(pyrrolidin-1-carbonyl)-phenyl]-amid},
(2R)-4-Pyrrolidin-1,2-dicarbonsäure-1-[(4-ethinyl-phenyl)-amid]-2-{[4-(pyrrolidin-1-carbonyl)-phenyl]-amid},
(2R,4R)-4-Methoxy-pyrrolidin-1,2-dicarbonsäure-1-[(4-ethinyl-phenyl)-amid]-2-{[4-(pyrrolidin-1-carbonyl)-phenyl]-amid},
(2R,4R)-4-Ethoxy-pyrrolidin-1,2-dicarbonsäure-1-[(4-ethinyl-phenyl)-amid]-2-{[4-(pyrrolidin-1-carbonyl)-phenyl]-amid},
(2R,4R)-4-Hydroxy-pyrrolidin-1,2-dicarbonsäure-1-[(4-ethinyl-phenyl)-amid]-2-{[2-fluor-4-(pyrrolidin-1-carbonyl)-phenyl]-amid},
(2R)-Pyrrolidin-1,2-dicarbonsäure-1-[(4-ethinyl-phenyl)-amid]-2-{[2-fluor-4-(pyrrolidin-1-carbonyl)-phenyl]-amid},
(2R,4R)-4-Methoxy-pyrrolidin-1,2-dicarbonsäure-1-[(4-ethinyl-phenyl)-amid]-2-{[2-fluor-4-(pyrrolidin-1-carbonyl)-phenyl]-amid},
(2R,4R)-4-Ethoxy-pyrrolidin-1,2-dicarbonsäure-1-[(4-ethinyl-phenyl)-amid]-2-{[2-fluor-4-(pyrrolidin-1-carbonyl)-phenyl]-amid}.

### Beispiel 3

### Analog Beispiel 1 erhält man die nachstehenden Verbindungen

(2R,4R)-4-Hydroxy-pyrrolidin-1,2-dicarbonsäure-1-[(4-ethinyl-phenyl)-amid]-2-{[2-fluor-4-(5-methyl-pyrazol-1-yl)-phenyl]-amid},
(2R)-Pyrrolidin-1,2-dicarbonsäure-1-[(4-ethinyl-phenyl)-amid]-2-{[2-fluor-4-(5-methyl-pyrazol-1-yl)-phenyl]-amid},
(2R,4R)-4-Methoxy-pyrrolidin-1,2-dicarbonsäure-1-[(4-ethinyl-phenyl)-amid]-2-{[2-fluor-4-(5-methy)-pyrazol-1-yl)-phenyl]-amid},
(2R,4R)-4-Ethoxy-pyrrolidin-1,2-dicarbonsäure-1-[(4-ethinyl-phenyl)-amid]-2-{[2-fluor-4-(5-methyl-pyrazol-1-yl)-phenyl]-amid},
(2R,4R)-4-Hydroxy-pyrrolidin-1,2-dicarbonsäure-1-[(4-ethinyl-phenyl)-amid]-2-{[2-fluor-4-(3-methyl-pyrazol-1-yl)-phenyl]-amid},
(2R,4R)-Pyrrolidin-1,2-dicarbonsäure-1-[(4-ethinyl-phenyl)-amid]-2-{[2-fluor-4-(3-methyl-pyrazol-1-yl)-phenyl]-amid},
(2R,4R)-4-Methoxy-pyrrolidin-1,2-dicarbonsäure-1-[(4-ethinyl-phenyl)-amid]-2-{[2-fluor-4-(3-methyl-pyrazol-1-yl)-phenyl]-amid},
(2R,4R)-4-Ethoxy-pyrrolidin-1,2-dicarbonsäure-1-[(4-ethinyl-phenyl)-amid]-2-{[2-fluor-4-(3-methyl-pyrazol-1-yl)-phenyl]-amid}.

### Beispiel 4

### Analog Beispiel 1 erhält man die nachstehenden Verbindungen

(2R,4R)-4-Ethoxy-pyrrolidin-1,2-dicarbonsäure-1-[(4-ethinyl-phenyl)-amid]-2-{[4-(2-oxo-piperidin-1-yl)-phenyl]-amid} ("A4-1 "),
(2R,4R)-4-Ethoxy-pyrrolidin-1,2-dicarbonsäure-1-[(4-ethinyl-phenyl)-amid]-2-{[3-fluor-4-(2-oxo-2*H*-pyridin-1-yl)-phenyl]-amid} ("A4-2"),
(2R,4R)-4-Ethoxy-pyrrolidin-1,2-dicarbonsäure-1-[(4-ethinyl-phenyl)-amid]-2-{[4-(3-oxo-morpholin-4-yl)-phenyl]-amid},
(2S,4R)-4-Ethoxy-pyrrolidin-1,2-dicarbonsäure-1-[(4-ethinyl-phenyl)-amid]-2-{[4-(3-oxo-morpholin-4-yl)-phenyl]-amid},
(2R,4R)-4-Ethoxy-pyrrolidin-1,2-dicarbonsäure-1-((4-ethinyl-phenyl)-amid]-2-{[4-(2-oxo-2*H*-pyrazin-1-yl)-phenyl]-amid},
(2S,4R)-4-Ethoxy-pyrrolidin-1,2-dicarbonsäure-1-[(4-ethinyl-phenyl)-amid]-2-{[4-(2-oxo-2*H*- pyrazin-1-yl)-phenyl]-amid},
(2R,4R)-4-Hydroxy-pyrrolidin-1,2-dicarbonsäure-1-[(4-ethinyl-phenyl)-amid]-2-{[4-(2-oxo-piperidin-1-yl)-phenyl]-amid},
(2R,4R)-4-Hydroxy-pyrrolidin-1,2-dicarbonsäure-1-[(4-ethinyl-phenyl)-amid]-2-{[4-(2-oxo-pyrrolidin-1 -yl)-phenyl]-amid},
(2R,4R)-4-Hydroxy-pyrrolidin-1,2-dicarbonsäure-1-[(4-ethinyl-phenyl)-amid]-2-{[3-methyl-4-(2-oxo-piperidin-1-yl)-phenyl]-amid},
(2R,4R)-4-Hydroxy-pyrrolidin-1,2-dicarbonsäure-1-[(4-ethinyl-phenyl)-amid]-2-{[3-methyl-4-(2-oxo-pyrrolidin-1-yl)-phenyl]-amid},
(2R,4R)-4-Ethoxy-pyrrolidin-1,2-dicarbonsäure-1-[(4-ethinyl-phenyl)-amid]-2-{[3-fluor-4-(3-oxo-morpholin-4-yl)-phenyl]-amid},
(2S,4R)-4-Ethoxy-pyrrolidin-1,2-dicarbonsäure-1-[(4-ethinyl-phenyl)-amid]-2-{[3-fluor-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[1-acetyl-2,3-dihydro-1*H*-indol-5-yl]-amid} ("A4-3"),
(2R,4R)-4-Hydroxy-pyrrolidin-1,2-dicarbonsäure-1-[(4-ethinyl-phenyl)-amid]-2-{[2-ethoxycarbonyl-1*H*-indol-5-yl]-amid},
(2R,4R)-4-Hydroxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Methoxy-pyrrolidin-1,2-dicarbonsäure-1-[(4-ethinyl-phenyl)-amid]-2-{[3-methoxy-4-(2-oxo-2*H*-pyridin-1-yl)-phenyl]-amid} ("A4-4"),
(2R,4R)-4-Hydroxy-pyrrolidin-1,2-dicarbonsäure-1-[(4-ethinyl-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Allyloxy-pyrrolidin-1,2-dicarbonsäure-1-[(4-ethinyl-phenyl)-amid]-2-{[4-(3-oxo-morpholin-4-yl)-phenyl]-amid} ("A4-5"),
(2R,4R)-4-Allyloxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Propargyloxy-pyrrolidin-1,2-dicarbonsäure-1-[(4-ethinyl-phenyl)-amid]-2-{[4-(3-oxo-morpholin-4-yl)-phenyl]-amid} ("A4-6"),
(2R,4R)-4-Propargyloxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Methoxy-pyrrolidin-1,2-dicarbonsäure-1-[(4-ethinyl-phenyl)-amid]-2-{[2-fluor-4-(2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[4-(3-methyl-2-oxo-2*H*-pyridin-1-yl)-phenyl]-amid},
(2R,4S)-4-Propargyloxy-pyrrolidin-1,2-dicarbonsäure-1-[(4-ethinyl-phenyl)-amid]-2-([2-fluor-4-(3-oxo-morpholin-4-yl)-phenyl]-amid),
(2R,4R)-4-(2,3-Dihydroxy-propoxy)-pyrrolidin-1,2-dicarbonsäure-1-[(4-ethinyl-phenyl)-amid]-2-{[4-(3-oxo-morpholin-4-yl)-phenyl]-amid} ("A4-7"),
(2R,4R)-4-Methoxy-pyrrolidin-1,2-dicarbonsäure-1-[(4-ethinyl-phenyl)-amid]-2-{[4-(5-methyl-2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[4-(2-methoxycarbonyl-4-hydroxy-pyrrolidin-1-yl)-phenyl]-amid} ("A4-8"), F. 103°;
(2S,4R)-4-Methoxy-pyrrolidin-1,2-dicarbonsäure-1-[(4-ethinyl-phenyl)-amid]-2-{[2-fluor-4-(3-methyl-2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[2-fluor-4-(3-methyl-2-oxo-2*H*-pyridin-1-yl)-phenyl]-amid},
(2R,4R)-4-(Methoxyethoxy)-pyrrolidin-1,2-dicarbonsäure-1-[(4-ethinyl-phenyl)-amid]-2-{[4-(3-oxo-morpholin-4-yl)-phenyl]-amid} ("A4-9"),
(2R,4R)-4-(Methoxycarbonylmethoxy)-pyrrolidin-1,2-dicarbonsäure-1-[(4-ethinyl-phenyl)-amid]-2-{[4-(3-oxo-morpholin-4-yl)-phenyl]-amid} ("A4-10"),
(2R,4R)-4-(Carboxymethoxy)-pyrrolidin-1,2-dicarbonsäure-1-[(4-ethinyl-phenyl)-amid]-2-{[4-(3-oxo-morpholin-4-yl)-phenyl]-amid} ("A4-11"),
(2R,4R)-4-Methoxy-pyrrolidin-1,2-dicarbonsäure-1-[(4-ethinyl-phenyl)-amid]-2-{[4-(6-methyl-3-oxo-2*H*-pyridazin-2-yl)-phenyl]-amid} ("A4-12"),
(2R,4R)-4-(Methoxyethoxy)-pyrrolidin-1,2-dicarbonsäure-1-[(4-ethinyl-phenyl)-amid]-2-{[2-methyl-4-(3-oxo-morpholin-4-yl)-phenyl]-amid},
(2R,4R)-4-(Methoxyethoxy)-pyrrolidin-1,2-dicarbonsäure-1-[(4-ethinyl-phenyl)-amid]-2-{[2-fluor-4-(3-oxo-morpholin-4-yl)-phenyl]-amid}.

### Pharmakologische Daten (Affinität zu Rezeptoren)

| Verbindung Nr. | FXa-IC₅₀ [M] | TF/FVIIa-IC₅₀ [M] |
|---|---|---|
| "A1" | 1.3 x 10⁻⁹ | 1.9 x 10⁻⁹ |
| "A2" | 3.1 x 10⁻⁹ | 3.2 x 10⁻⁹ |
| "A4-1" | 3.0 x 10⁻⁹ | |
| "A4-2" | 1.9 x 10⁻⁹ | |
| "A4-3" | 2.6 x 10⁻⁶ | |
| "A4-4" | 2.8 x 10⁻⁹ | |
| "A4-5" | 3.1 x 10⁻⁹ | |
| "A4-6" | 1.3 x 10⁻⁹ | |
| "A4-7" | 3.1 x 10⁻⁹ | |
| "A4-8" | 1.1 x 10⁻⁶ | |
| "A4-9" | 3.3 x 10⁻⁹ | |
| "A4-10" | 4.2 x 10⁻⁹ | |
| "A4-11" | 4.8 x 10⁻⁹ | |
| "A4-12" | 2.1 x 10⁻⁶ | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 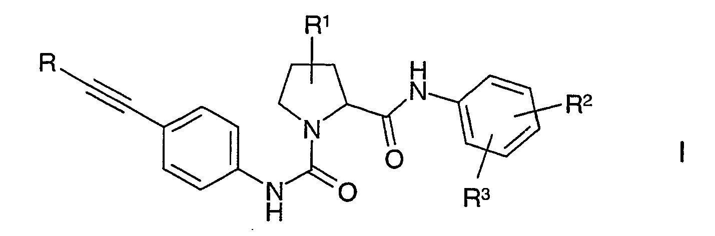 worin
R H, X, A, X-CO- oder A-CO-,
R¹ H, =O, Hal, X, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)ₘ-O-, -O(CH₂)ₘCOOH oder -O(CH₂)ₘOA,
R² H, Hal oder A,
R³ einen einkernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, CN, (CH₂)ₙOH, NR⁴R⁵, =NH, =N-OH, =N-OA, COOA und/oder Carbonylsauerstoff (=O) substituiert sein kann,
oder CONR⁴R⁵,
R² und R³ zusammen auch -CH=CH-NH- oder -CH₂-CH₂-NH, wobei ein H-Atom durch A-CO- oder A-O-CO- ersetzt sein kann,
R⁴, R⁵ unabhängig voneinander H oder A,
R⁴ und R⁵ zusammen auch eine Alkylenkette mit 3, 4 oder 5 C-Atomen, die auch durch A, Hal, OA und/oder Carbonylsauerstoff (=CO) substituiert sein kann,
X Aryl, Arylalkyl, Het oder Het-alkyl,
Aryl unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
Het einen ein- oder zweikernigen gesättigten, ungesättigten oder aromatischen Heterocyclus mit 1 bis 4 N-, O-und/oder S-Atomen, der unsubstituiert oder ein-, zwei- oder dreifach durch Hal, A, Benzyl, Cycloalkyl, OH, NH₂, NHCONH₂, NO₂, CN, -CH₂-COOH, -CH₂-CONH₂, NHCOA, NR³SO₂A, CHO, SO₂NH₂, SO₂A und/oder Carbonylsauerstoff substituiert sein kann,
A unverzweigtes, verzweigtes oder cylisches Alkyl mit 1-10 C-Atomen, worin auch 1-7 H-Atome durch F und/oder Chlor ersetzt sein können,
Hal F, Cl, Br oder I,
m 1, 2, 3, 4, 5 oder 6
n 0, 1, 2, 3, 4 , 5 oder 6
bedeuten,
sowie ihre pharmazeutisch verwendbaren Salze, Solvate und Stereoisomere, einschließlich deren Mischungen in allen Verhältnissen.

2. Verbindungen nach Anspruch 1, worin
R H oder A bedeutet.

3. Verbindungen gemäß Anspruch 1 oder 2,
worin
R³ einen einkernigen gesättigten, ungesättigten oder aromatischen Heterocyclus mit 1 bis 4 N-, O- und/oder S-Atomen, der unsubstituiert oder ein-, zwei- oder dreifach durch Hal, A, OA, =NH, OH, COOA und/oder Carbonylsauerstoff (=O) substituiert sein kann,
oder CONR⁴R⁵ bedeutet.

4. Verbindungen gemäß einem oder mehreren der Ansprüche 1-3, worin
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl, 4*H*-[1,4]Oxazin-4-yl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OA, OH, COOA und/oder A substituiert,
oder
CONR⁴R⁵,
R⁴, R⁵ unabhängig voneinander H oder A,
R⁴ und R⁵ zusammen auch eine Alkylenkette mit 3, 4 oder 5 C-Atomen,
bedeuten.

5. Verbindungen gemäß einem oder mehreren der Ansprüche 1-4, worin
R H, X, A, X-CO- oder A-CO-,
R¹ H, =O, Hal, X, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)ₘ-O-, -O(CH₂)ₘCOOH oder -O(CH₂)ₘOA,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2,6-Dioxo-piperidinl-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl, Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OA, OH, COOA und/oder A substituiert,
oder
CONR⁴R⁵,
R⁴, R⁵ unabhängig voneinander H oder A,
R⁴ und R⁵ zusammen auch eine Alkylenkette mit 3, 4 oder 5 C-Atomen,
X Aryl, Arylalkyl, Het oder Het-alkyl,
Aryl unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
Het einen ein- oder zweikernigen gesättigten, ungesättigten oder aromatischen Heterocyclus mit 1 bis 4 N-, O-und/oder S-Atomen, der unsubstituiert oder ein-, zwei- oder dreifach durch Hal, A, Benzyl, Cycloalkyl, OH, NH₂, NHCONH₂, NO₂, CN, -CH₂-COOH, -CH₂-CONH₂, NHCOA, NR³SO₂A, CHO, SO₂NH₂, SO₂A und/oder Carbonylsauerstoff substituiert sein kann,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten.

6. Verbindungen gemäß einem oder mehreren der Ansprüche 1-5,
worin
R H oder A,
R¹ H, OH, OA, O-Allyl, O-Propargyl, OCH₂CH(OH)CH₂OH, A-O-CO-(CH₂)ₘ-O-, -O(CH₂)ₘCOOH oder -O(CH₂)ₘOA,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl, 3-Oxo-2*H*-pyridazin-2-yl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl,
gegebenenfalls ein- oder zweifach durch Hal, OA, OH, COOA und/oder A substituiert,
oder CONR⁴R⁵,
R⁴ und R⁵ zusammen eine Alkylenkette mit 3, 4 oder 5 C-Atomen,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten.

7. Verbindungen gemäß einem oder mehreren der Ansprüche 1-6, worin
R H, X, A, X-CO- oder A-CO-,
R¹ H, =O, Hal, X, A, OH, OA, A-COO-, A-CONH-, A-CONA-, N₃, NH₂, NO₂, CN, COOH, COOA, CONH₂, CON(A)₂, O-Allyl, O-Propargyl, O-Benzyl, =N-OH, =N-OA, OCH₂CH(OH)CH₂OH, A-O-CO-(CH₂)ₘ-O-, -O(CH₂)ₘCOOH oder -O(CH₂)ₘOA,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Imino-piperidin-1-yl, 2-Imino-pyrrolidin-1-yl, 3-Imino-morpholin-4-yl, 2-Imino-imidazolidin-1-yl, 2-Imino-1*H*-Pyrazin-1-yl, 2,6-Dioxo-piperidin1-yl, 2-Oxo-piperazin-1-yl, 2,6-Dioxo-piperazin-1-yl, 2,5-Dioxo-pyrrolidin-1-yl, 2-Oxo-1,3-oxazolidin-3-yl, 3-Oxo-2*H*-pyridazin-2-yl, 2-Caprolactam-1-yl (= 2-Oxo-azepan-1-yl), 2-Aza-bicyclo[2.2.2]-octan-3-on-2-yl, 5,6-Dihydro-1*H*-pyrimidin-2-oxo-1-yl, 2-Oxo-[1,3]oxazinan-3-yl oder 4*H*-[1,4]Oxazin-4-yl,
X Aryl, Arylalkyl, Het oder Het-alkyl,
Aryl unsubstituiertes oder ein-, zwei- oder dreifach durch Hal, A, OH, NH₂, NO₂, CN, COOH, COOA, CONH₂, NHCOA, NHCONH₂, NHSO₂A, CHO, COA, SO₂NH₂, SO₂A, -CH₂-COOH oder -OCH₂-COOH substituiertes Phenyl, Naphthyl oder Biphenyl,
Het einen ein- oder zweikernigen gesättigten, ungesättigten oder aromatischen Heterocyclus mit 1 bis 4 N-, O-und/oder S-Atomen, der unsubstituiert oder ein-, zwei- oder dreifach durch Hal, A, Benzyl, Cycloalkyl, OH, NH₂, NHCONH₂, NO₂, CN, -CH₂-COOH, -CH₂-CONH₂, NHCOA, NR³SO₂A, CHO, SO₂NH₂, SO₂A und/oder Carbonylsauerstoff substituiert sein kann,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten,
sowie ihre pharmazeutisch verwendbaren Derivate, Salze, Solvate und Stereoisomere, einschließlich deren Mischungen in allen Verhältnissen.

8. Verbindungen gemäß einem oder mehreren der Ansprüche 1-7 , worin
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl,
bedeutet.

9. Verbindungen gemäß einem oder mehreren der Ansprüche 1-8, worin
R¹ H, OH, OA, O-Allyl, O-Propargyl, OCH₂CH(OH)CH₂OH, A-O-CO-(CH₂)ₘ-O-, -O(CH₂)ₘCOOH oder -O(CH₂)ₘOA,
bedeutet.

10. Verbindungen gemäß einem oder mehreren der Ansprüche 1-9, worin
A unverzweigtes oder verzweigtes Alkyl mit 1-6 C-Atomen bedeutet,

11. Verbindungen gemäß einem oder mehreren der Ansprüche 1-10, worin
R H oder A,
R¹ H, OH, OA, O-Allyl, O-Propargyl, OCH₂CH(OH)CH₂OH, A-O-CO-(CH₂)ₘ-O-, -O(CH₂)ₘCOOH oder -O(CH₂)ₘOA,
R² H, Hal oder A,
R³ 2-Oxo-piperidin-1-yl, 2-Oxo-pyrrolidin-1-yl, 2-Oxo-1*H-*pyridin-1-yl, 3-Oxo-morpholin-4-yl, 4-Oxo-1*H*-pyridin-1-yl, 2-Oxo-1*H*-Pyrazin-1-yl, 2-Oxo-imidazolidin-1-yl, 2-Oxo-piperazin-1-yl oder 3-Oxo-2*H*-pyridazin-2-yl, gegebenenfalls einfach durch A, OH, COOA substituiert,
A unverzweigtes, verzweigtes oder cylisches Alkyl mit 1-10 C-Atomen, worin auch 1-7 H-Atome durch F ersetzt sein können,
Hal F, Cl, Br oder I,
bedeuten.

12. Verbindungen gemäß Anspruch 1
(2R,4R)-4-Methoxy-pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-([4-(3-oxo-morpholin-4-yl)-phenyl]-amid),
(2R,4R)-4-Methoxy-pyrrolidin-1,2-dicarbonsäure-1 -[(4-ethinyl-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[4-(2-oxo-*2H*-pyridin-1-yl)-phenyl]-amid},
(2R,4R)-4-Hydroxy-pyrrolidin-1,2-dicarbonsäure-1-[(4-ethinyl-phenyl)-amid]-2-{[3-methyl-4-(3-oxo-morpholin-4-yl)-phenyl]-amid},
(2R)-Pyrrolidin-1,2-dicarbonsäure-1-[(4-ethinyl-phenyl)-amid]-2-{[4-(3-oxo-morpholin-4-yl)-phenyl]-amid},
(2R)-Pyrrolidin-1,2-dicarbonsäure-1-[(4-ethinyl-phenyl)-amid]-2-{[3-methyl-4-(3-oxo-morpholin-4-yl)-phenyl]-amid},
(2R)-Pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Ethoxy-pyrrolidin-1,2-dicarbonsäure-1-[(4-ethinyl-phenyl)-amid]-2-{[3-methyl-4-(3-oxo-morpholin-4-yl)-phenyl]-amid},
(2R)-Pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[4-(2-oxo-1*H*-pyrazin-1-yl)-phenyl]-amid},
(2R,4R)-4-Ethoxy-pyrrolidin-1,2-dicarbonsäure-1-[(4-ethinyl-phenyl)-amid]-2-{[4-(2-oxo-piperidin-1-yl)-phenyl]-amid},
(2R,4R)-4-Ethoxy-pyrrolidin-1,2-dicarbonsäure-1-[(4-ethinyl-phenyl)-amid]-2-{[3-fluor-4-(2-oxo-2*H*-pyridin-1-yl)-phenyl]-amid},
(2R,4R)-4-Ethoxy-pyrrolidin-1,2-dicarbonsäure-1-[(4-ethinyl-phenyl)-amid]-2-{[4-(3-oxo-morpholin-4-yl)-phenyl]-amid},
(2S,4R)-4-Ethoxy-pyrrolidin-1,2-dicarbonsäure-1-[(4-ethinyl-phenyl)-amid]-2-{[4-(3-oxo-morpholin-4-yl)-phenyl]-amid},
(2R,4R)-4-Ethoxy-pyrrolidin-1,2-dicarbonsäure-1-[(4-ethinyl-phenyl)-amid]-2-{[4-(2-oxo-2*H*-pyrazin-1-yl)-phenyl]-amid},
(2S,4R)-4-Ethoxy-pyrrolidin-1,2-dicarbonsäure-1-[(4-ethinyl-phenyl)-amid]-2-{[4-(2-oxo-2*H*-pyrazin-1-yl)-phenyl]-amid},
(2R,4R)-4-Hydroxy-pyrrolidin-1,2-dicarbonsäure-1-[(4-ethinyl-phenyl)-amid]-2-{[4-(2-oxo-piperidin-1-yl)-phenyl]-amid},
(2R,4R)-4-Hydroxy-pyrrolidin-1,2-dicarbonsäure-1-[(4-ethinyl-phenyl)-amid]-2-{[4-(2-oxo-pyrrolidin-1-yl)-phenyl]-amid},
(2R,4R)-4-Hydroxy-pyrrolidin-1,2-dicarbonsäure-1-[(4-ethinyl-phenyl)-amid]-2-{[3-methyl-4-(2-oxo-piperidin-1-yl)-phenyl]-amid},
(2R,4R)-4-Hydroxy-pyrrolidin-1,2-dicarbonsäure-1-[(4-ethinyl-phenyl)-amid]-2-{[3-methyl-4-(2-oxo-pyrrolidin-1-yl)-phenyl]-amid},
(2R,4R)-4-Ethoxy-pyrrolidin-1,2-dicarbonsäure-1-[(4-ethinyl-phenyl)-amid]-2-{[3-fluor-4-(3-oxo-morpholin-4-yl)-phenyl]-amid},
(2S,4R)-4-Ethoxy-pyrrolidin-1,2-dicarbonsäure-1-[(4-ethinyl-phenyl)-amid]-2-{[3-fluor-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[1-acetyl-2,3-dihydro-1*H*-indol-5-yl]-amid},
(2R,4R)-4-Hydroxy-pyrrolidin-1,2-dicarbonsäure-1-[(4-ethinyl-phenyl)-amid]-2-{[2-ethoxycarbonyl-1*H*-indol-5-yl]-amid},
(2R,4R)-4-Hydroxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Methoxy-pyrrolidin-1,2-dicarbonsäure-1-[(4-ethinyl-phenyl)-amid]-2-{[3-methoxy-4-(2-oxo-2*H*-pyridin-1-yl)-phenyl]-amid},
(2R,4R)-4-Hydroxy-pyrrolidin-1,2-dicarbonsäure-1-[(4-ethinyl-phenyl)-amid]-2-{[3-methyl-4-(3-oxo-morpholin-4-yl)-phenyl]-amid},
(2R,4R)-4-Hydroxy-pyrrolidin-1,2-dicarbonsäure-1-[(4-ethinyl-phenyl)-amid]-2-{[4-(2-oxo-2*H*-pyridin-1-yl)-phenyl]-amid},
(2R,4R)-4-Allyloxy-pyrrolidin-1,2-dicarbonsäure-1-[(4-ethinyl-phenyl)-amid]-2-{[4-(3-oxo-morpholin-4-yl)-phenyl]-amid},
(2R,4R)-4-Allyloxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Propargyloxy-pyrrolidin-1,2-dicarbonsäure-1 -[(4-ethinyl-phenyl)-amid]-2-{[4-(3-oxo-morpholin-4-yl)-phenyl]-amid},
(2R,4R)-4-Propargyloxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-Methoxy-pyrrolidin-1,2-dicarbonsäure-1 -[(4-ethinyl-phenyl)-amid]-2-{[2-fluor-4-(2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[4-(3-methyl-2-oxo-2*H*-pyridin-1-yl)-phenyl]-amid},
(2R,4S)-4-Propargyloxy-pyrrolidin-1,2-dicarbonsäure-1-[(4-ethinyl-phenyl)-amid]-2-{[2-fluor-4-(3-oxo-morpholin-4-yl)-phenyl]-amid},
(2R,4R)-4-(2,3-Dihydroxy-propoxy)-pyrrolidin-1,2-dicarbonsäure-1-[(4-ethinyl-phenyl)-amid]-2-{[4-(3-oxo-morpholin-4-yl)-phenyl]-amid},
(2R,4R)-4-Methoxy-pyrrolidin-1,2-dicarbonsäure-1 -[(4-ethinyl-phenyl)-amid]-2-{[4-(5-methyl-2-oxo-2H pyridin-1-yl)-phenyl]-amid},
(2R,4R)-4-Methoxy-pyrrolidin-1,2-dicarbonsäure-1-[(4-ethinyl-phenyl)-amid]-2-{[4-(2-methoxycarbonyl-4-hydroxy-pyrrolidin-1-yl)-phenyl]-amid},
(2S,4R)-4-Methoxy-pyrrolidin-1,2-dicarbonsäure-1-[(4-ethinyl-phenyl)-amid]-2-{[2-fluor-4-(3-methyl-2-oxo-2*H*-pyridin-1-yl)-phenyl]-amid},
(2R,4R)-4-Methoxy-pyrrolidin-1,2-dicarbonsäure-1-[(4-ethinyl-phenyl)-amid]-2-{[2-fluor-4-(3-methyl-2-oxo-2*H*-pyridin-1-yl)-phenyl]-amid},
(2R,4R)-4-(Methoxyethoxy)-pyrrolidin-1,2-dicarbonsäure-1-[(4-ethinyl-phenyl)-amid]-2-{[4-(3-oxo-morpholin-4-yl)-phenyl]-amid},
(2R,4R)-4-(Methoxycarbonylmethoxy)-pyrrolidin-1,2-dicarbonsäure-1-[(4-ethinyl-phenyl)-amid]-2-{[4-(3-oxo-morpholin-4-yl)-phenyl]-amid},
(2R,4R)-4-(Carboxymethoxy)-pyrrolidin-1,2-dicarbonsäure-1-[(4-ethinyl-phenyl)-amid]-2-{[4-(3-oxo-morpholin-4-yl)-phenyl]-amid},
(2R,4R)-4-Methoxy-pyrrolidin-1,2-dicarbonsäure-1-[(4-ethinyl-phenyl)-amid]-2-{[4-(6-methyl-3-oxo-2*H*-pyridazin-2-yl)-phenyl]-amid},
(2R,4R)-4-(Methoxyethoxy)-pyrrolidin-1,2-dicarbonsäure-1-[(4-ethinyl-phenyl)-amid]-2-{[2-methyl-4-(3-oxo-morpholin-4-yl)-phenyl]-amid},
(2R,4R)-4-(Methoxyethoxy)-pyrrolidin-1,2-dicarbonsäure-1-[(4-ethinyl-phenyl)-amid]-2-{[2-fluor-4-(3-oxo-morpholin-4-yl)-phenyl]-amid}.

13. Verfahren zur Herstellung von Verbindungen der Formel I nach den Ansprüchen 1-7, **dadurch gekennzeichnet, daß** man
a) eine Verbindung der Formel II 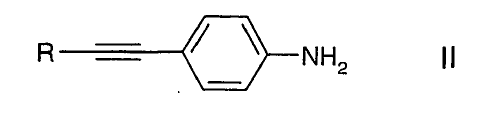 worin R die in Anspruch 1 angegebene Bedeutung hat,
mit einem Chloroformiatderivat zu einem intermediären Carbamatderivat umsetzt,
das anschließend mit einer Verbindung der Formel III 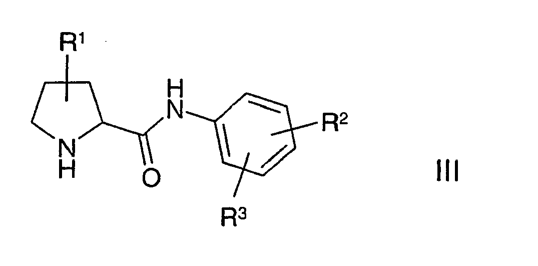 worin
R¹, R² und R³ die in Anspruch 1 angegebene Bedeutung haben,
umgesetzt wird,
oder
b) eine Verbindung der Formel III
mit einer Verbindung der Formel IV 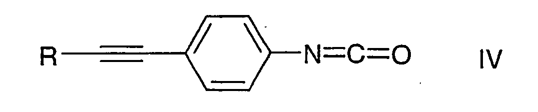 worin
R die in Anspruch 1 angegebene Bedeutung hat,
umsetzt,
oder
c) eine Verbindung der Formel V 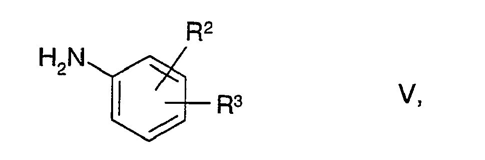 worin R² und R³ die in Anspruch 1 angegebene Bedeutung haben,
mit einer Verbindung der Formel VI 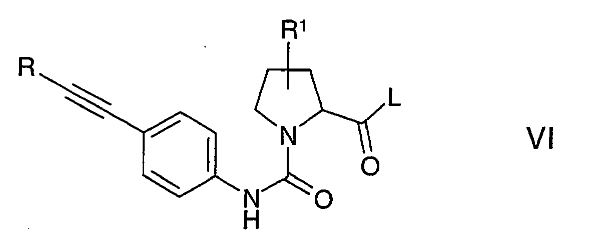
worin
L Cl, Br, I oder eine freie oder reaktionsfähig funktionell abgewandelte OH-Gruppe bedeutet und
R und R¹ die in Anspruch 1 angegebenen Bedeutungen haben,
umsetzt,
und/oder eine Base oder Säure der Formel I in eines ihrer Salze umwandelt.

14. Verbindungen der Formel I nach einem oder mehreren der Ansprüche 1 bis 12 als Inhibitoren des Koagulationsfaktors Xa.

15. Verbindungen der Formel I nach einem oder mehreren der Ansprüche 1 bis 12 als Inhibitoren des Koagulationsfaktors Vlla.

16. Arzneimittel, enthaltend mindestens eine Verbindung der Formel I nach einem oder mehreren der Ansprüche 1 bis 12 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

17. Arzneimittel enthaltend mindestens eine Verbindung der Formel I gemäß einem oder mehreren der Ansprüche 1 bis 12 und/oder ihre pharmazeutisch verwendbaren Salze, Solvate und Stereoisomere, einschließlich deren Mischungen in allen Verhältnissen, und mindestens einen weiteren Arzneimittelwirkstoff.

18. Verwendung von Verbindungen gemäß einem oder mehreren der Ansprüche 1 bis 12 und/oder ihre physiologisch unbedenklichen Salze und Solvate zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tinnitus, Tumoren, Tumorerkrankungen und/oder Tumormetastasen.

19. Set (Kit), bestehend aus getrennten Packungen von
(a) einer wirksamen Menge an einer Verbindung der Formel I gemäß einem oder mehreren der Ansprüche 1 bis 12 und/oder ihrer pharmazeutisch verwendbaren Salze, Solvate und Stereoisomere, einschließlich deren Mischungen in allen Verhältnissen, und
(b) einer wirksamen Menge eines weiteren Arzneimittelswirkstoffs.

20. Verwendung von Verbindungen der Formel I gemäß einem oder mehreren der Ansprüche 1 bis 12 und/oder ihrer pharmazeutisch verwendbaren Salze, Solvate und Stereoisomere, einschließlich deren Mischungen in allen Verhältnissen, zur Herstellung eines Arzneimittels zur Behandlung von Thrombosen, myocardialem Infarkt, Arteriosklerose, Entzündungen, Apoplexie, Angina pectoris, Restenose nach Angioplastie, Claudicatio intermittens, Migräne, Tinnitus, Tumoren, Tumorerkrankungen und/oder Tumormetastasen,
in Kombination mit mindestens einem weiteren Arzneimittelwirkstoff.

## Claims

1. Compounds of the formula I 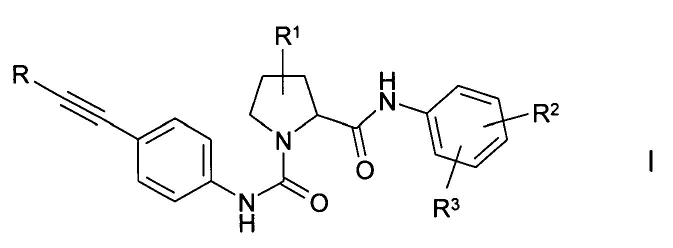 in which
R denotes H, X, A, X-CO- or A-CO-,
R¹ denotes H, =O, Hal, X, A, OH, OA, A-COO-, A-CONH-, A-CONA-, N₃, NH₂, NO₂, CN, COOH, COOA, CONH₂, CON(A)₂, O-allyl, O-propargyl, O-benzyl, =N-OH, =N-OA, OCH₂CH(OH)CH₂OH, A-O-CO-(CH₂)ₘ-O-, -O(CH₂)mCOOH or -O(CH₂)ₘOA,
R² denotes H, Hal or A,
R³ denotes a monocyclic saturated, unsaturated or aromatic heterocyclic radical having 1 to 4 N, O and/or S atoms, which may be unsubstituted or mono-, di- or trisubstituted by Hal, A, OA, CN, (CH₂)ₙOH, NR⁴R⁵, =NH, =N-OH, =N-OA, COOA and/or carbonyl oxygen (=O), or CONR⁴R⁵,
R² and R³ together also denote -CH=CH-NH- or -CH₂-CH₂-NH, where one H atom may be replaced by A-CO- or A-O-CO-,
R⁴, R⁵, independently of one another, denote H or A,
R⁴ and R⁵ together also denote an alkylene chain having 3, 4 or 5 C atoms, which may also be substituted by A, Hal, OA and/or carbonyl oxygen (=CO),
X denotes aryl, arylalkyl, Het or Het-alkyl,
aryl denotes phenyl, naphthyl or biphenyl, each of which is unsubstituted or mono-, di- or trisubstituted by Hal, A, OH, NH₂, NO₂, CN, COOH, COOA, CONH₂, NHCOA, NHCONH₂, NHSO₂A, CHO, COA, SO₂NH₂, SO₂A, -CH₂-COOH or -OCH₂-COOH,
Het denotes a mono- or bicyclic saturated, unsaturated or aromatic heterocyclic radical having 1 to 4 N, O and/or S atoms, which may be unsubstituted or mono-, di- or trisubstituted by Hal, A, benzyl, cycloalkyl, OH, NH₂, NHCONH₂, NO₂, CN, -CH₂-COOH, -CH₂-CONH₂, NHCOA, NR³SO₂A, CHO, SO₂NH₂, SO₂A and/or carbonyl oxygen,
A denotes unbranched, branched or cyclic alkyl having 1-10 C atoms, in which, in addition, 1-7 H atoms may be replaced by F and/or chlorine,
Hal denotes F, Cl, Br or I,
m denotes 1, 2, 3, 4, 5 or 6,
n denotes 0, 1, 2, 3, 4 , 5 or 6,
and pharmaceutically usable salts, solvates and stereoisomers thereof, including mixtures thereof in all ratios.

2. Compounds according to Claim 1 in which
R denotes H or A.

3. Compounds according to Claim 1 or 2
in which
R³ denotes a monocyclic saturated, unsaturated or aromatic heterocyclic radical having 1 to 4 N, O and/or S atoms, which may be unsubstituted or mono-, di- or trisubstituted by Hal, A, OA, =NH, OH, COOA and/or carbonyl oxygen (=O),
or CONR⁴R⁵.

4. Compounds according to one or more of Claims 1-3
in which
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-imino-piperidin-1-yl, 2-iminopyrrolidin-1-yl, 3-iminomorpholin-4-yl, 2-iminoimidazolidin-1-yl, 2-imino-1*H*-pyrazin-1-yl, 2,6-dioxopiperidin-1-yl, 2-oxopiperazin-1-yl, 2,6-dioxopiperazin-1-yl, 2,5-dioxopyrrolidin-1-yl, 2-oxo-1,3-oxazolidin-3-yl, 3-oxo-2*H*-pyridazin-2-yl, 2-caprolactam-1-yl (= 2-oxoazepan-1-yl), 2-azabicyclo[2.2.2]octan-3-on-2-yl, 5,6-dihydro-1*H*-pyrimidin-2-oxo-1-yl, 2-oxo-1,3-oxazinan-3-yl, 4*H*-1,4-oxazin-4-yl,
furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl, optionally mono- or disubstituted by Hal, OA, OH, COOA and/or A,
or
CONR⁴R⁵,
R⁴, R⁵, independently of one another, denote H or A,
R⁴ and R⁵ together also denote an alkylene chain having 3, 4 or 5 C atoms.

5. Compounds according to one or more of Claims 1-4
in which
R denotes H, X, A, X-CO- or A-CO-,
R¹ denotes H, =O, Hal, X, A, OH, OA, A-COO-, A-CONH-, A-CONA-, N₃, NH₂, NO₂, CN, COOH, COOA, CONH₂, CON(A)₂, O-allyl, O-propargyl, O-benzyl, =N-OH, =N-OA, OCH₂CH(OH)CH₂OH, A-O-CO-(CH₂)ₘ-O-, -O(CH₂)ₘCOOH or -O(CH₂)ₘOA,
R² denotes H, Hal or A,
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-iminopiperidin-1-yl, 2-iminopyrrolidin-1-yl, 3-iminomorpholin-4-yl, 2-iminoimidazolidin-1-yl, 2-imino-1*H*-pyrazin-1-yl, 2,6-dioxopiperidin-1-yl, 2-oxopiperazin-1-yl, 2,6-dioxopiperazin-1-yl, 2,5-dioxopyrrolidin-1-yl, 2-oxo-1,3-oxazolidin-3-yl, 3-oxo-2H-pyridazin-2-yl, 2-caprolactam-1-yl (= 2-oxoazepan-1-yl), 2-azabicyclo[2.2.2]octan-3-on-2-yl, 5,6-dihydro-1*H*-pyrimidin-2-oxo-1-yl, 2-oxo-1,3-oxazinan-3-yl or 4*H*-1,4-oxazin-4-yl,
furyl, thienyl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl,
optionally mono- or disubstituted by Hal, OA, OH, COOA and/or A,
or
CONR⁴R⁵,
R⁴, R⁵, independently of one another, denote H or A,
R⁴ and R⁵ together also denote an alkylene chain having 3, 4 or 5 C atoms,
X denotes aryl, arylalkyl, Het or Het-alkyl,
aryl denotes phenyl, naphthyl or biphenyl, each of which is unsubstituted or mono-, di- or trisubstituted by Hal, A, OH, NH₂, NO₂, CN, COOH, COOA, CONH₂, NHCOA, NHCONH₂, NHSO₂A, CHO, COA, SO₂NH₂, SO₂A, -CH₂-COOH or -OCH₂-COOH,
Het denotes a mono- or bicyclic saturated, unsaturated or aromatic heterocyclic radical having 1 to 4 N, O and/or S atoms, which may be unsubstituted or mono-, di- or trisubstituted by Hal, A, benzyl, cycloalkyl, OH, NH₂, NHCONH₂, NO₂, CN, -CH₂-COOH, -CH₂-CONH₂, NHCOA, NR³SO₂A, CHO, SO₂NH₂, SO₂A and/or carbonyl oxygen,
A denotes unbranched, branched or cyclic alkyl having 1-10 C atoms, in which, in addition, 1-7 H atoms may be replaced by F,
Hal denotes F, Cl, Br or I.

6. Compounds according to one or more of Claims 1-5
in which
R denotes H or A,
R¹ denotes H, OH, OA, O-allyl, O-propargyl,
OCH₂CH(OH)CH₂OH, A-O-CO-(CH₂)ₘ-O-, -O(CH₂)ₘCOOH or -O(CH₂)ₘOA,
R² denotes H, Hal or A,
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-oxo-piperazin-1-yl, 3-oxo-2*H*-pyridazin-2-yl, pyrrolyl, imidazolyl, pyrazolyl, oxazolyl, isoxazolyl, thiazolyl, isothiazolyl, pyridyl, pyrimidinyl, triazolyl, tetrazolyl, oxadiazolyl, thiadiazolyl, pyridazinyl, pyrazinyl,
optionally mono- or disubstituted by Hal, OA, OH, COOA and/or A,
or CONR⁴R⁵,
R⁴ and R⁵ together denote an alkylene chain having 3, 4 or 5 C atoms,
A denotes unbranched, branched or cyclic alkyl having 1-10 C atoms, in which, in addition, 1-7 H atoms may be replaced by F,
Hal denotes F, Cl, Br or I.

7. Compounds according to one or more of Claims 1-6
in which
R denotes H, X, A, X-CO- or A-CO-,
R¹ denotes H, =O, Hal, X, A, OH, OA, A-COO-, A-CONH-, A-CONA-, N₃, NH₂, NO₂, CN, COOH, COOA, CONH₂, CON(A)₂, O-allyl, O-propargyl, O-benzyl, =N-OH, =N-OA,
OCH₂CH(OH)CH₂OH, A-O-CO-(CH₂)ₘ-O-, -O(CH₂)ₘCOOH or -O(CH₂)ₘOA,
R² denotes H, Hal or A,
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-imino-piperidin-1-yl, 2-iminopyrrolidin-1-yl, 3-iminomorpholin-4-yl, 2-iminoimidazolidin-1-yl, 2-imino-1*H*-pyrazin-1-yl, 2,6-dioxopiperidin-1-yl, 2-oxopiperazin-1-yl, 2,6-dioxopiperazin-1-yl, 2,5-dioxopyrrolidin-1-yl, 2-oxo-1,3-oxazolidin-3-yl, 3-oxo-2*H*-pyridazin-2-yl, 2-caprolactam-1-yl (= 2-oxoazepan-1-yl), 2-azabicyclo[2.2.2]octan-3-on-2-yl, 5,6-dihydro-1*H*-pyrimidin-2-oxo-1-yl, 2-oxo-1,3-oxazinan-3-yl or 4*H*-1,4-oxazin-4-yl,
X denotes aryl, arylalkyl, Het or Het-alkyl,
aryl denotes phenyl, naphthyl or biphenyl, each of which is unsubstituted or mono-, di- or trisubstituted by Hal, A, OH, NH₂, NO₂, CN, COOH, COOA, CONH₂, NHCOA, NHCONH₂, NHSO₂A, CHO, COA, SO₂NH₂, SO₂A, -CH₂-COOH or -OCH₂-COOH,
Het denotes a mono- or bicyclic saturated, unsaturated or aromatic heterocyclic radical having 1 to 4 N, O and/or S atoms, which may be unsubstituted or mono-, di- or trisubstituted by Hal, A, benzyl, cycloalkyl, OH, NH₂, NHCONH₂, NO₂, CN, -CH₂-COOH, -CH₂-CONH₂, NHCOA, NR³SO₂A, CHO, SO₂NH₂, SO₂A and/or carbonyl oxygen,
A denotes unbranched, branched or cyclic alkyl having 1-10 C atoms, in which, in addition, 1-7 H atoms may be replaced by F,
Hal denotes F, Cl, Br or I,
and pharmaceutically usable derivatives, salts, solvates and stereoisomers thereof, including mixtures thereof in all ratios.

8. Compounds according to one or more of Claims 1-7
in which
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-oxo-piperazin-1-yl or 3-oxo-2H-pyridazin-2-yl.

9. Compounds according to one or more of Claims 1-8
in which
R¹ denotes H, OH, OA, O-allyl, O-propargyl,
OCH₂CH(OH)CH₂OH, A-O-CO-(CH₂)ₘ-O-, -O(CH₂)ₘCOOH or -O(CH₂)ₘOA.

10. Compounds according to one or more of Claims 1-9
in which
A denotes unbranched or branched alkyl having 1-6 C atoms.

11. Compounds according to one or more of Claims 1-10
in which
R denotes H or A,
R¹ denotes H, OH, OA, O-allyl, O-propargyl,
OCH₂CH(OH)CH₂OH, A-O-CO-(CH₂)ₘ-O-, -O(CH₂)ₘCOOH or -O(CH₂)ₘOA,
R² denotes H, Hal or A,
R³ denotes 2-oxopiperidin-1-yl, 2-oxopyrrolidin-1-yl, 2-oxo-1*H-*pyridin-1-yl, 3-oxomorpholin-4-yl, 4-oxo-1*H*-pyridin-1-yl, 2-oxo-1*H*-pyrazin-1-yl, 2-oxoimidazolidin-1-yl, 2-oxo-piperazin-1-yl or 3-oxo-2H-pyridazin-2-yl, optionally monosubstituted by A, OH, COOA,
A denotes unbranched, branched or cyclic alkyl having 1-10 C atoms, in which, in addition, 1-7 H atoms may be replaced by F,
Hal denotes F, Cl, Br or I.

12. Compounds according to Claim 1
N1-(4-ethynylphenyl)-N2-[4-(2-oxo-2*H*-pyridin-1-yl)phenyl]-(2R,4R)-4-methoxypyrrolidine-1,2-dicarboxamide,
N1-(4-ethynylphenyl)-N2-[4-(3-oxomorpholin-4-yl)phenyl]-(2R,4R)-4-methoxypyrrolidine-1,2-dicarboxamide,
N1-(4-ethynylphenyl)-N2-[3-methyl-4-(3-oxomorpholin-4-yl)-phenyl]-(2R,4R)-4-methoxypyrrolidine-1,2-dicarboxamide,
N1-(4-ethynylphenyl)-N2-[4-(3-oxomorpholin-4-yl)phenyl]-(2R,4R)-4-hydroxypyrrolidine-1,2-dicarboxamide,
N1-(4-ethynylphenyl)-N2-[3-methyl-4-(3-oxomorpholin-4-yl)-phenyl]-(2R,4R)-4-ethoxypyrrolidine-1,2-dicarboxamide,
N1-(4-ethynylphenyl)-N2-[4-(2-oxo-2*H*-pyridin-1-yl)phenyl]-(2R,4R)-4-hydroxypyrrolidine-1,2-dicarboxamide,
N1-(4-ethynylphenyl)-N2-[3-methyl-4-(3-oxomorpholin-4-yl)-phenyl]-(2R,4R)-4-hydroxypyrrolidine-1,2-dicarboxamide,
N1-(4-ethynylphenyl)-N2-[4-(3-oxomorpholin-4-yl)phenyl]-(2R)-pyrrolidine-1,2-dicarboxamide,
N1-(4-ethynylphenyl)-N2-[3-methyl-4-(3-oxomorpholin-4-yl)-phenyl]-(2R)-pyrrolidine-1,2-dicarboxamide,
N1-(4-ethynylphenyl)-N2-[4-(2-oxo-2*H*-pyridin-1-yl)phenyl]-(2R)-pyrrolidine-1,2-dicarboxamide,
N1-(4-ethynylphenyl)-N2-[2-fluoro-4-(3-oxomorpholin-4-yl)-phenyl]-(2R,4R)-4-methoxypyrrolidine-1,2-dicarboxamide,
N1-(4-ethynylphenyl)-N2-[4-(2-oxo-2*H*-pyridin-1-yl)phenyl]-(2R,4R)-4-ethoxypyrrolidine-1,2-dicarboxamide,
N1-(4-ethynylphenyl)-N2-[3-methyl-4-(3-oxomorpholin-4-yl)-phenyl]-(2R,4R)-4-ethoxypyrrolidine-1,2-dicarboxamide,
N1-(4-ethynylphenyl)-N2-[2-fluoro-4-(3-oxomorpholin-4-yl)-phenyl]-(2R)-pyrrolidine-1,2-dicarboxamide,
N1-(4-ethynylphenyl)-N2-[2-fluoro-4-(3-oxomorpholin-4-yl)-phenyl]-(2R,4R)-4-ethoxypyrrolidine-1,2-dicarboxamide,
N1-(4-ethynylphenyl)-N2-[2-fluoro-4-(3-oxomorpholin-4-yl)-phenyl]-(2R,4R)-4-hydroxypyrrolidine-1,2-dicarboxamide,
N1-(4-ethynylphenyl)-N2-[4-(2-oxo-1*H*-pyrazin-1-yl)phenyl]-(2R,4R)-4-ethoxypyrrolidine-1,2-dicarboxamide,
N1-(4-ethynylphenyl)-N2-[4-(2-oxopiperidin-1-yl)phenyl]-(2R,4R)-4-ethoxypyrrolidine-1,2-dicarboxamide,
N1-(4-ethynylphenyl)-N2-[3-fluoro-4-(2-oxo-2*H*-pyridin-1-yl)-phenyl]-(2R,4R)-4-ethoxypyrrolidine-1,2-dicarboxamide,
N1-(4-ethynylphenyl)-N2-[4-(3-oxomorpholin-4-yl)phenyl]-(2R,4R)-4-ethoxypyrrolidine-1,2-dicarboxamide,
N1-(4-ethynylphenyl)-N2-[4-(3-oxomorpholin-4-yl)phenyl]-(2S,4R)-4-ethoxypyrrolidine-1,2-dicarboxamide,
N1-(4-ethynylphenyl)-N2-[4-(2-oxo-2*H*-pyrazin-1-yl)phenyl]-(2R,4R)-4-ethoxypyrrolidine-1,2-dicarboxamide,
N1-(4-ethynylphenyl)-N2-[4-(2-oxo-2*H*-pyrazin-1-yl)phenyl]-(2S,4R)-4-ethoxypyrrolidine-1,2-dicarboxamide,
N1-(4-ethynylphenyl)-N2-[4-(2-oxopiperidin-1-yl)phenyl]-(2R,4R)-4-hydroxypyrrolidine-1,2-dicarboxamide,
N1-(4-ethynylphenyl)-N2-[4-(2-oxopyrrolidin-1-yl)phenyl]-(2R,4R)-4-hydroxypyrrolidine-1,2-dicarboxamide,
N1-(4-ethynylphenyl)-N2-[3-methyl-4-(2-oxopiperidin-1-yl)-phenyl]-(2R,4R)-4-hydroxypyrrolidine-1,2-dicarboxamide,
N1-(4-ethynylphenyl)-N2-[3-methyl-4-(2-oxopyrrolidin-1-yl)-phenyl]-(2R,4R)-4-hydroxypyrrolidine-1,2-dicarboxamide,
N1-(4-ethynylphenyl)-N2-[3-fluoro-4-(3-oxomorpholin-4-yl)-phenyl]-(2R,4R)-4-ethoxypyrrolidine-1,2-dicarboxamide,
N1-(4-ethynylphenyl)-N2-[3-fluoro-4-(3-oxomorpholin-4-yl)-phenyl]-(2S,4R)-4-ethoxypyrrolidine-1,2-dicarboxamide,
N1-(4-ethynylphenyl)-N2-[1-acetyl-2,3-dihydro-1*H*-indol-5-yl]-(2R,4R)-4-hydroxypyrrolidine-1,2-dicarboxamide,
N1-(4-ethynylphenyl)-N2-[2-ethoxycarbonyl-1*H*-indol-5-yl]-(2R,4R)-4-hydroxypyrrolidine-1,2-dicarboxamide,
N1-(4-ethynylphenyl)-N2-[2-fluoro-4-(3-oxomorpholin-4-yl)-phenyl]-(2R,4R)-4-hydroxypyrrolidine-1,2-dicarboxamide,
N1-(4-ethynylphenyl)-N2-[3-methoxy-4-(2-oxo-2*H*-pyridin-1-yl)-phenyl]-(2R,4R)-4-methoxypyrrolidine-1,2-dicarboxamide,
N1-(4-ethynylphenyl)-N2-[3-methyl-4-(3-oxomorpholin-4-yl)-phenyl]-(2R,4R)-4-hydroxypyrrolidine-1,2-dicarboxamide,
N1-(4-ethynylphenyl)-N2-[4-(2-oxo-2*H*-pyridin-1-yl)phenyl]-(2R,4R)-4-hydroxypyrrolidine-1,2-dicarboxamide,
N1-(4-ethynylphenyl)-N2-[4-(3-oxomorpholin-4-yl)phenyl]-(2R,4R)-4-allyloxypyrrolidine-1,2-dicarboxamide,
N1-(4-ethynylphenyl)-N2-[2-fluoro-4-(3-oxomorpholin-4-yl)-phenyl]-(2R,4R)-4-allyloxypyrrolidine-1,2-dicarboxamide,
N1-(4-ethynylphenyl)-N2-[4-(3-oxomorpholin-4-yl)phenyl]-(2R,4R)-4-propargyloxypyrrolidine-1,2-dicarboxamide,
N1-(4-ethynylphenyl)-N2-[2-fluoro-4-(3-oxomorpholin-4-yl)-phenyl]-(2R,4R)-4-propargyloxypyrrolidine-1,2-dicarboxamide,
N1-(4-ethynylphenyl)-N2-[2-fluoro-4-(2-oxo-2*H*-pyridin-1-yl)-phenyl]-(2R,4R)-4-methoxypyrrolidine-1,2-dicarboxamide,
N1-(4-ethynylphenyl)-N2-[4-(3-methyl-2-oxo-2*H*-pyridin-1-yl)-phenyl]-(2R,4R)-4-methoxypyrrolidine-1,2-dicarboxamide,
N1-(4-ethynylphenyl)-N2-[2-fluoro-4-(3-oxomorpholin-4-yl)-phenyl]-(2R,4S)-4-propargyloxypyrrolidine-1,2-dicarboxamide,
N1-(4-ethynylphenyl)-N2-[4-(3-oxomorpholin-4-yl)phenyl]-(2R,4R)-4-(2,3-dihydroxypropoxy)pyrrolidine-1,2-dicarboxamide,
N1-(4-ethynylphenyl)-N2-[4-(5-methyl-2-oxo-2*H*-pyridin-1-yl)-phenyl]-(2R,4R)-4-methoxypyrrolidine-1,2-dicarboxamide,
N1-(4-ethynylphenyl)-N2-[4-(2-methoxycarbonyl-4-hydroxypyrrolidin-1-yl)phenyl]-(2R,4R)-4-methoxypyrrolidine-1,2-dicarboxamide,
N1-(4-ethynylphenyl)-N2-[2-fluoro-4-(3-methyl-2-oxo-2*H*-pyridin-1-yl)phenyl]-(2S,4R)-4-methoxypyrrolidine-1,2-dicarboxamide,
N1-(4-ethynylphenyl)-N2-[2-fluoro-4-(3-methyl-2-oxo-2*H*-pyridin-1-yl)phenyl]-(2R,4R)-4-methoxypyrrolidine-1,2-dicarboxamide,
N1-(4-ethynylphenyl)-N2-[4-(3-oxomorpholin-4-yl)phenyl]-(2R,4R)-4-(methoxyethoxy)pyrrolidine-1,2-dicarboxamide,
N1-(4-ethynylphenyl)-N2-[4-(3-oxomorpholin-4-yl)phenyl]-(2R,4R)-4-(methoxycarbonylmethoxy)pyrrolidine-1,2-dicarboxamide,
N1-(4-ethynylphenyl)-N2-[4-(3-oxomorpholin-4-yl)phenyl]-(2R,4R)-4-(carboxymethoxy)pyrrolidine-1,2-dicarboxamide,
N1-(4-ethynylphenyl)-N2-[4-(6-methy)-3-oxo-2*H*-pyridazin-2-yl)phenyl]-(2R,4R)-4-methoxypyrrolidine-1,2-dicarboxamide,
N1-(4-ethynylphenyl)-N2-[2-methyl-4-(3-oxomorpholin-4-yl)-phenyl]-(2R,4R)-4-(methoxyethoxy)pyrrolidine-1,2-dicarboxamide,
N1-(4-ethynylphenyl)-N2-[2-fluoro-4-(3-oxomorpholin-4-yl)-phenyl]-(2R,4R)-4-(methoxyethoxy)pyrrolidine-1,2-dicarboxamide.

13. Process for the preparation of compounds of the formula I according to Claims 1-7, **characterised in that**
a) a compound of the formula II 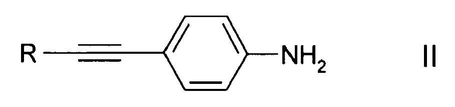 in which R has the meaning indicated in Claim 1,
is reacted with a chloroformate derivative to give a carbamate derivative intermediate,
which is subsequently reacted with a compound of the formula III 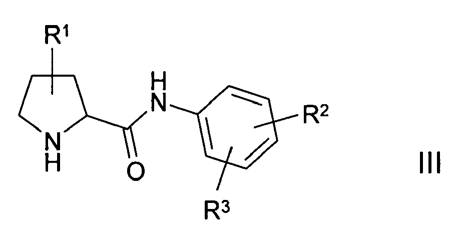 in which
R¹, R² and R³ have the meaning indicated in Claim 1,
or
b) a compound of the formula III
is reacted with a compound of the formula IV 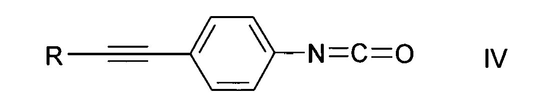 in which
R has the meaning indicated in Claim 1,
or
c) a compound of the formula V 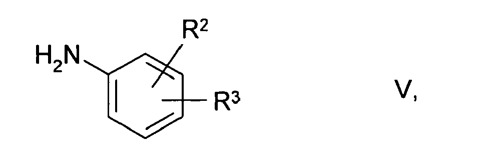 in which R² and R³ have the meaning indicated in Claim 1,
is reacted with a compound of the formula VI 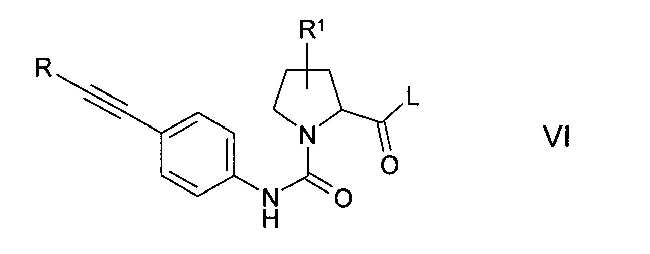
in which
L denotes Cl, Br, I or a free or reactively functionally modified OH group, and
R and R¹ have the meanings indicated in Claim 1,
and/or a base or acid of the formula I is converted into one of its salts.

14. Compounds of the formula I according to one or more of Claims 1 to 12 as inhibitors of coagulation factor Xa.

15. Compounds of the formula I according to one or more of Claims 1 to 12 as inhibitors of coagulation factor Vlla.

16. Medicaments comprising at least one compound of the formula I according to one or more of Claims 1 to 12 and/or pharmaceutically usable salts, solvates and stereoisomers thereof, including mixtures thereof in all ratios, and optionally excipients and/or adjuvants.

17. Medicaments comprising at least one compound of the formula I according to one or more of Claims 1 to 12 and/or pharmaceutically usable salts, solvates and stereoisomers thereof, including mixtures thereof in all ratios, and at least one further medicament active ingredient.

18. Use of compounds according to one or more of Claims 1 to 12 and/or physiologically acceptable salts and solvates thereof for the preparation of a medicament for the treatment of thromboses, myocardial infarction, arteriosclerosis, inflammations, apoplexy, angina pectoris, restenosis after angioplasty, claudicatio intermittens, migraine, tinnitus, tumours, tumour diseases and/or tumour metastases.

19. Set (kit) consisting of separate packs of
(a) an effective amount of a compound of the formula I according to one or more of Claims 1 to 12 and/or pharmaceutically usable salts, solvates and stereoisomers thereof, including mixtures thereof in all ratios,
and
(b) an effective amount of a further medicament active ingredient.

20. Use of compounds of the formula I according to one or more of Claims 1 to 12 and/or pharmaceutically usable salts, solvates and stereoisomers thereof, including mixtures thereof in all ratios, for the preparation of a medicament for the treatment of thromboses, myocardial infarction, arteriosclerosis, inflammations, apoplexy, angina pectoris, restenosis after angioplasty, claudicatio intermittens, migraine, tinnitus, tumours, tumour diseases and/or tumour metastases,
in combination with at least one further medicament active ingredient.

## Revendications

1. Composés de la formule I 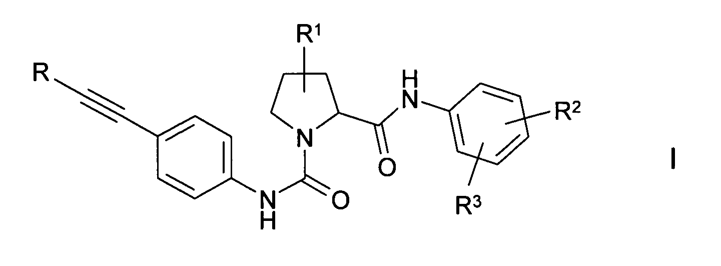 dans laquelle
R représente H, X, A, X-CO- ou A-CO-,
R¹ représente H, =O, Hal, X, A, OH, OA, A-COO-, A-CONH-, A-CONA-, N₃, NH₂, NO₂, CN, COOH, COOA, CONH₂, CON(A)₂, O-allyle, O-propargyle, O-benzyle, =N-OH, =N-OA, OCH₂CH(OH)CH₂OH, A-O-CO-(CH₂)ₘ-O-, -O(CH₂)ₘCOOH ou -O(CH₂)ₘOA,
R² représente H, Hal ou A,
R³ représente un radical hétérocyclique saturé, non saturé ou aromatique monocyclique comportant de 1 à 4 atomes de N, O et/ou S, qui peut être non substitué ou mono-, di- ou trisubstitué par Hal, A, OA, CN, (CH₂)ₙOH, NR⁴R⁵, =NH, =N-OH, =N-OA, COOA et/ou oxygène carbonyle (=O), ou CONR⁴R⁵,
R² et R³ représentent également ensemble -CH=CH-NH- ou -CH₂₋CH₂-NH où un atome de H peut être remplacé par A-CO- ou A-O-CO-,
R⁴ R⁵ indépendamment l'un de l'autre, représentent H ou A,
R⁴ et R⁵ représentent également ensemble une chaîne alkylène comportant 3, 4 ou 5 atomes de C, qui peut également être substituée par A, Hal, OA et/ou oxygène carbonyle (=CO),
X représente aryle, arylalkyle, Het ou Het-alkyle,
aryle représente phényle, naphthyle ou diphényle dont chacun est non substitué ou mono-, di- ou trisubstitué par Hal, A, OH, NH₂, NO₂, CN, COOH, COOA, CONH₂, NHCOA, NHCONH₂, NHSO₂A, CHO, COA, SO₂NH₂, SO₂A, -CH₂-COOH ou -OCH₂-COOH,
Het représente un radical hétérocyclique saturé, non saturé ou aromatique mono- ou bicyclique comportant de 1 à 4 atomes de N, O et/ou S, qui peut être non substitué ou mono-, di- ou trisubstitué par Hal, A, benzyle, cycloalkyle, OH, NH₂, NHCONH₂, NO₂, CN, -CH₂-COOH, -CH₂-CONH₂, NHCOA, NR³SO₂A, CHO, SO₂NH₂, SO₂A et/ou oxygène carbonyle,
A représente un alkyle non ramifié, ramifié ou cyclique comportant de 1 à 10 atomes de C où, en plus, de 1 à 7 atomes de H peuvent être remplacés par F et/ou chlore,
Hal représente F, Cl, Br ou I,
m représente 1, 2, 3, 4, 5 ou 6,
n représente 0, 1, 2, 3, 4, 5 ou 6,
et leurs sels, solvates et stéréo-isomères pharmaceutiquement utilisables, y compris des mélanges afférents selon tous rapports.

2. Composés selon la revendication 1, dans lesquels R représente H ou A.

3. Composés selon la revendication 1 ou 2, dans lesquels
R³ représente un radical hétérocyclique saturé, non saturé ou aromatique monocyclique comportant de 1 à 4 atomes de N, O et/ou S, qui peut être non substitué ou mono-, di- ou tri-substitué par Hal, A, OA, =NH, OH, COOA et/ou oxygène carbonyle (=O),
ou CONR⁴R⁵.

4. Composés selon une ou plusieurs des revendications 1 à 3, dans lesquels
R³ représente 2-oxopipéridine-1-yle, 2-oxopyrrolidine-1-yle, 2-oxo-1*H*-pyridine-1-yle, 3-oxomorpholine-4-yle, 4-oxo-1*H-*pyridine-1-yle, 2-oxo-1*H*-pyrazine-1-yle, 2-oxoimidazolidine-1-yle, 2-iminopipéridine-1-yle, 2-iminopyrrolidine-1-yle, 3-iminomorpholine-4-yle, 2-iminoimidazolidine-1-yle, 2-imino-1*H*-pyrazine-1-yle, 2,6-dioxopipéridine-1-yle, 2-oxopipérazine-1-yle, 2,6-dioxopipérazine-1-yle, 2,5-dioxopyrrolidine-1-yle, 2-oxo-1,3-oxazolidine-3-yle, 3-oxo-2*H*-pyridazine-2-yle, 2-caprolactam-1-yle (= 2-oxoazepan-1-yle), 2-azabicyclo-[2.2.2]octan-3-on-2-yle, 5,6-dihydro-1*H*-pyrimidine-2-oxo-1-yle, 2-oxo-1,3-oxazinan-3-yle, 4*H*-1,4-oxazine-4-yle, furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle,
en option mono- ou disubstitué par Hal, OA, OH, COOA et/ou A,
ou
CONR⁴R⁵,
R⁴, R⁵ indépendamment l'un de l'autre, représentent H ou A,
R⁴ et R⁵ représentent également ensemble une chaîne alkylène comportant 3, 4 ou 5 atomes de C.

5. Composés selon une ou plusieurs des revendications 1 à 4, dans lesquels
R représente H, X, A, X-CO- ou A-CO-,
R¹ représente H, =O, Hal, X, A, OH, OA, A-COO-, A-CONH-, A-CONA-, N₃, NH₂, NO₂, CN, COOH, COOA, CONH₂, CON(A)₂, O-allyle, O-propargyle, O-benzyle, =N-OH, =N-OA, OCH₂CH(OH)CH₂OH, A-O-CO-(CH₂)m-O-, -O(CH₂)ₘCOOH ou -O(CH₂)ₘOA,
R² représente H, Hal ou A,
R³ représente 2-oxopipéridine-1-yle, 2-oxopyrrolidine-1-yle, 2-oxo-1*H*-pyridine-1-yle, 3-oxomorpholine-4-yle, 4-oxo-1*H-*pyridine-1-yle, 2-oxo-1*H*-pyrazine-1-yle, 2-oxoimidazolidine-1-yle, 2-iminopipéridine-1-yle, 2-iminopyrrolidine-1-yle, 3-iminomorpholine-4-yle, 2-iminoimidazolidine-1-yle, 2-imino-1*H*-pyrazine-1-yle, 2,6-dioxopipéridine-1-yle, 2-oxopipérazine-1-yle, 2,6-dioxopipérazine-1-yle, 2,5-dioxopyrrolidine-1-yle, 2-oxo-1,3-oxazolidine-3-yle, 3-oxo-2H-pyridazine-2-yle, 2-caprolactam-1-yle (= 2-oxoazepan-1-yle), 2-azabicyclo-[2.2.2]octan-3-on-2-yle, 5,6-dihydro-1*H*-pyrimidine-2-oxo-1-yle, 2-oxo-1,3-oxazinan-3-yle ou 4*H*-1,4-oxazine-4-yle, furyle, thiényle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle,
en option mono- ou disubstitué par Hal, OA, OH, COOA et/ou A,
ou
CONR⁴R⁵,
R⁴, R⁵ indépendamment l'un de l'autre, représentent H ou A,
R⁴ et R⁵ représentent également ensemble une chaîne alkylène comportant 3, 4 ou 5 atomes de C,
X représente aryle, arylalkyle, Het ou Het-alkyle,
aryle représente phényle, naphthyle ou diphényle dont chacun est non substitué ou mono-, di- ou trisubstitué par Hal, A, OH, NH₂, NO₂, CN, COOH, COOA, CONH₂, NHCOA, NHCONH₂, NHSO₂A, CHO, COA, SO₂NH₂, SO₂A, -CH₂-COOH ou -OCH₂-COOH,
Het représente un radical hétérocyclique saturé, non saturé ou aromatique mono- ou bicyclique comportant de 1 à 4 atomes de N, O et/ou S, qui peut être non substitué ou mono-, di- ou trisubstitué par Hal, A, benzyle, cycloalkyle, OH, NH₂, NHCONH₂, NO₂, CN, -CH₂-COOH, -CH₂-CONH₂, NHCOA, NR³SO₂A, CHO, SO₂NH₂, SO₂A et/ou oxygène carbonyle
A représente un alkyle non ramifié, ramifié ou cyclique comportant de 1 à 10 atomes de C où, en plus, de 1 à 7 atomes de H peuvent être remplacés par F,
Hal représente F, CI, Br ou I.

6. Composés selon une ou plusieurs des revendications 1 à 5, dans lesquels
R représente H ou A,
R¹ représente H, OH, OA, O-allyle, O-propargyle, OCH₂CH(OH)CH₂OH, A-O-CO-(CH₂)ₘ-O-, -O(CH₂)mCOOH ou -O(CH₂)ₘOA,
R² représente H, Hal ou A,
R³ représente 2-oxopipéridine-1-yle, 2-oxopyrrolidine-1-yle, 2-oxo-1*H*-pyridine-1-yle, 3-oxomorpholine-4-yle, 4-oxo-1*H-*pyridine-1-yle, 2-oxo-1*H*-pyrazine-1-yle, 2-oxoimidazolidine-1-yle, 2-oxopipérazine-1-yle, 3-oxo-2*H*-pyridazine-2-yle, pyrrolyle, imidazolyle, pyrazolyle, oxazolyle, isoxazolyle, thiazolyle, isothiazolyle, pyridyle, pyrimidinyle, triazolyle, tétrazolyle, oxadiazolyle, thiadiazolyle, pyridazinyle, pyrazinyle,
en option mono- ou disubstitué par Hal, OA, OH, COOA et/ou A,
ou CONR⁴R⁵,
R⁴ et R⁵ représentent ensemble une chaîne alkylène comportant 3, 4 ou 5 atomes de C,
A représente un alkyle non ramifié, ramifié ou cyclique comportant de 1 à 10 atomes de C où, en plus, de 1 à 7 atomes de H peuvent être remplacés par F,
Hal représente F, CI, Br ou I.

7. Composés selon une ou plusieurs des revendications 1 à 6, dans lesquels
R représente H, X, A, X-CO- ou A-CO-,
R¹ représente H, =O, Hal, X, A, OH, OA, A-COO-, A-CONH-, A-CONA-, N₃, NH₂, NO₂, CN, COOH, COOA, CONH₂, CON(A)₂, O-allyle, O-propargyle, O-benzyle, =N-OH, =N-OA, OCH₂CH(OH)CH₂OH, A-O-CO-(CH₂)ₘ-O-, -O(CH₂)ₘCOOH ou -O(CH₂)ₘOA,
R² représente H, Hal ou A,
R³ représente 2-oxopipéridine-1-yle, 2-oxopyrrolidine-1-yle, 2-oxo-1*H*-pyridine-1-yle, 3-oxomorpholine-4-yle, 4-oxo-1*H-*pyridine-1-yle, 2-oxo-1*H*-pyrazine-1-yle, 2-oxoimidazolidine-1-yle, 2-iminopipéridine-1-yle, 2-iminopyrrolidine-1-yle, 3-iminomorpholine-4-yle, 2-iminoimidazolidine-1-yle, 2-imino-1*H*-pyrazine-1-yle, 2,6-dioxopipéridine-1-yle, 2-oxopipéra-zine-1-yle, 2,6-dioxopipérazine-1-yle, 2,5-dioxopyrrolidine-1-yle, 2-oxo-1,3-oxazolidine-3-yle, 3-oxo-2*H*-pyridazine-2-yle, 2-caprolactam-1-yle (= 2-oxoazepan-1-yle), 2-azabicyclo-[2.2.2]octan-3-on-2-yle, 5,6-dihydro-1*H*-pyrimidine-2-oxo-1-yle, 2-oxo-1,3-oxazinan-3-yle ou 4*H*-1,4-oxazine-4-yle,
X représente aryle, arylalkyle, Het ou Het-alkyle,
aryle représente phényle, naphthyle ou biphényle, dont chacun est non substitué ou mono-, di- ou trisubstitué par Hal, A, OH, NH₂, NO₂, CN, COOH, COOA, CONH₂, NHCOA, NHCONH₂, NHSO₂A, CHO, COA, SO₂NH₂, SO₂A, -CH₂-COOH ou -OCH₂-COOH,
Het représente un radical hétérocyclique saturé, non saturé ou aromatique mono- ou bicyclique comportant de 1 à 4 atomes de N, O et/ou S, qui peut être non substitué ou mono-, di- ou trisubstitué par Hal, A, benzyle, cycloalkyle, OH, NH₂, NHCONH₂, NO₂, CN, -CH₂-COOH, -CH₂-CONH₂, NHCOA, NR³SO₂A, CHO, SO₂NH₂, SO₂A et/ou oxygène carbonyle
A représente un alkyle non ramifié, ramifié ou cyclique comportant de 1 à 10 atomes de C où, en plus, de 1 à 7 atomes de H peuvent être remplacés par F,
Hal représente F, Cl, Br ou I,
et leurs dérivés, sels, solvates et stéréo-isomères pharmaceutiquement utilisables, y compris des mélanges afférents selon tous rapports.

8. Composés selon une ou plusieurs des revendications 1 à 7, dans lesquels
R³ représente 2-oxopipéridine-1-yle, 2-oxopyrrolidine-1-yle, 2-oxo-1*H*-pyridine-1-yle, 3-oxomorpholine-4-yle, 4-oxo-1*H-*pyridine-1-yle, 2-oxo-1*H*-pyrazine-1-yle, 2-oxoimidazolidine-1-yle, 2-oxopipérazine-1-yle ou 3-oxo-2H-pyridazine-2-yle.

9. Composés selon une ou plusieurs des revendications 1 à 8, dans lesquels
R¹ représente H, OH, OA, O-allyle, O-propargyle, OCH₂CH(OH)CH₂OH, A-O-CO-(CH₂)ₘ-O-, -O(CH₂)ₘCOOH ou -O(CH₂)ₘOA.

10. Composés selon une ou plusieurs des revendications 1 à 9, dans lesquels
A représente un alkyle non ramifié ou ramifié comportant de 1 à 6 atomes de C.

11. Composés selon une ou plusieurs des revendications 1 à 10, dans lesquels
R représente H ou A,
R¹ représente H, OH, OA, O-allyle, O-propargyle, OCH₂CH(OH)CH₂OH, A-O-CO-(CH₂)ₘ-O-, -O(CH₂)ₘCOOH ou -O(CH₂)ₘOA,
R² représente H, Hal ou A,
R³ représente 2-oxopipéridine-1-yle, 2-oxopyrrolidine-1-yle, 2-oxo-1*H*-pyridine-1-yle, 3-oxomorpholine-4-yle, 4-oxo-1*H-*pyridine-1-yle, 2-oxo-1*H*-pyrazine-1-yle, 2-oxoimidazolidine-1-yle, 2-oxopipérazine-1-yle ou 3-oxo-2*H*-pyridazine-2-yle, en option monosubstitué par A, OH, COOA,
A représente un alkyle non ramifié, ramifié ou cyclique comportant de 1 à 10 atomes de C où, en plus, de 1 à 7 atomes de H peuvent être remplacés par F,
Hal représente F, Cl, Br ou I.

12. Composés selon la revendication 1,
N1-(4-éthynylphényl)-N2-[4-(2-oxo-2*H*-pyridine-1-yl)phényl]-(2R,4R)-4-méthoxypyrrolidine-1,2-dicarboxamide,
N1-(4-éthynylphényl)-N2-[4-(3-oxomorpholine-4-yl)phényl]-(2R,4R)-4-méthoxypyrrolidine-1,2-dicarboxamide,
N1-(4-éthynylphényl)-N2-[3-methyl-4-(3-oxomorpholine-4-yl)-phényl]-(2R,4R)-4-méthoxypyrrolidine-1,2-dicarboxamide,
N1-(4-éthynylphényl)-N2-[4-(3-oxomorpholine-4-yl)phényl]-(2R,4R)-4-hydroxypyrrolidine-1,2-dicarboxamide,
N1-(4-éthynylphényl)-N2-[3-méthyl-4-(3-oxomorpholine-4-yl)-phényl]-(2R,4R)-4-éthoxypyrrolidine-1,2-dicarboxamide,
N1-(4-éthynylphényl)-N2-[4-(2-oxo-2*H*-pyridine-1-yl)phényl]-(2R,4R)-4-hydroxypyrrolidine-1,2-dicarboxamide,
N1-(4-éthynylphényl)-N2-[3-méthyl-4-(3-oxomorpholine-4-yl)-phényl]-(2R,4R)-4-hydroxypyrrolidine-1,2-dicarboxamide,
N1-(4-éthynylphényl)-N2-[4-(3-oxomorpholine-4-yl)phényl]-(2R)-pyrrolidine-1,2-dicarboxamide,
N1-(4-éthynylphényl)-N2-[3-méthyl-4-(3-oxomorpholine-4-yl)-phényl]-(2R)-pyrrolidine-1,2-dicarboxamide,
N1-(4-éthynylphényl)-N2-[4-(2-oxo-2*H*-pyridine-1-yl)phényl]-(2R)-pyrrolidine-1,2-dicarboxamide,
N1-(4-éthynylphényl)-N2-[2-fluoro-4-(3-oxomorpholine-4-yl)-phényl]-(2R,4R)-4-méthoxypyrrolidine-1,2-dicarboxamide,
N1-(4-éthynylphényl)-N2-[4-(2-oxo-2*H*-pyridine-1-yl)phényl]-(2R,4R)-4-éthoxypyrrolidine-1,2-dicarboxamide,
N1-(4-éthynylphényl)-N2-[3-méthyl-4-(3-oxomorpholine-4-yl)-phényl]-(2R,4R)-4-éthoxypyrrolidine-1,2-dicarboxamide,
N1-(4-éthynylphényl)-N2-[2-fluoro-4-(3-oxomorpholine-4-yl)-phényl]-(2R)-pyrrolidine-1,2-dicarboxamide,
N1-(4-éthynylphényl)-N2-[2-fluoro-4-(3-oxomorpholine-4-yl)-phényl]-(2R,4R)-4-éthoxypyrrolidine-1,2-dicarboxamide,
N1-(4-éthynylphényl)-N2-[2-fluoro-4-(3-oxomorpholine-4-yl)-phényl]-(2R,4R)-4-hydroxypyrrolidine-1,2-dicarboxamide,
N1-(4-éthynylphényl)-N2-[4-(2-oxo-1*H*-pyrazine-1-yl)phényl]-(2R,4R)-4-éthoxypyrrolidine-1,2-dicarboxamide,
N1-(4-éthynylphényl)-N2-[4-(2-oxopipéridine-1-yl)phényl]-(2R,4R)-4-éthoxypyrrolidine-1,2-dicarboxamide,
N1-(4-éthynylphényl)-N2-[3-fluoro-4-(2-oxo-2*H*-pyridine-1-yl)-phényl]-(2R,4R)-4-éthoxypyrrolidine-1,2-dicarboxamide,
N1-(4-éthynylphényl)-N2-[4-(3-oxomorpholine-4-yl)phényl]-(2R,4R)-4-éthoxypyrrolidine-1,2-dicarboxamide,
N1-(4-éthynylphényl)-N2-[4-(3-oxomorpholine-4-yl)phényl]-(2S,4R)-4-éthoxypyrrolidine-1,2-dicarboxamide,
N1-(4-éthynylphényl)-N2-[4-(2-oxo-2*H*-pyrazine-1-yl)phényl]-(2R,4R)-4-éthoxypyrrolidine-1,2-dicarboxamide,
N1-(4-éthynylphényl)-N2-[4-(2-oxo-2*H*-pyrazine-1-yl)phényl]-(2S,4R)-4-éthoxypyrrolidine-1,2-dicarboxamide,
N1-(4-éthynylphényl)-N2-[4-(2-oxopiperidine-1-yl)phényl]-(2R,4R)-4-hydroxypyrrolidine-1,2-dicarboxamide,
N1-(4-éthynylphényl)-N2-[4-(2-oxopyrrolidine-1-yl)phényl]-(2R,4R)-4-hydroxypyrrolidine-1,2-dicarboxamide,
N1-(4-éthynylphényl)-N2-[3-méthyl-4-(2-oxopipéridine-1-yl)-phényl]-(2R,4R)-4-hydroxypyrrolidine-1,2-dicarboxamide,
N1-(4-éthynylphényl)-N2-[3-méthyl-4-(2-oxopyrrolidine-1-yl)-phényl]-(2R,4R)-4-hydroxypyrrolidine-1,2-dicarboxamide,
N1-(4-éthynylphényl)-N2-[3-fluoro-4-(3-oxomorpholine-4-yl)-phényl]-(2R,4R)-4-éthoxypyrrolidine-1,2-dicarboxamide,
N1-(4-éthynylphényl)-N2-[3-fluoro-4-(3-oxomorpholine-4-yl)-phényl]-(2S,4R)-4-éthoxypyrrolidine-1,2-dicarboxamide,
N1-(4-éthynylphényl)-N2-[1-acétyl-2,3-dihydro-1*H*-indol-5-yl]-(2R,4R)-4-hydroxypyrrolidine-1,2-dicarboxamide,
N1-(4-éthynylphényl)-N2-[2-éthoxycarbonyl-1*H*-indol-5-yl]-(2R,4R)-4-hydroxypyrrolidine-1,2-dicarboxamide,
N1-(4-éthynylphényl)-N2-[2-fluoro-4-(3-oxomorpholine-4-yl)-phényl]-(2R,4R)-4-hydroxypyrrolidine-1,2-dicarboxamide,
N1-(4-éthynylphényl)-N2-[3-méthoxy-4-(2-oxo-2*H*-pyridine-1 -yl)-phényl]-(2R,4R)-4-méthoxypyrrolidine-1,2-dicarboxamide,
N -(4-éthynylphényl)-N2-[3-méthyl-4-(3-oxomorpholine-4-yl)-phényl]-(2R,4R)-4-hydroxypyrrolidine-1,2-dicarboxamide,
N1-(4-éthynylphényl)-N2-[4-(2-oxo-2*H*-pyridine-1-yl)phényl]-(2R,4R)-4-hydroxypyrrolidine-1,2-dicarboxamide,
N1-(4-éthynylphényl)-N2-[4-(3-oxomorpholine-4-yl)phényl]-(2R,4R)-4-allyloxypyrrolidine-1,2-dicarboxamide,
N1-(4-éthynylphényl)-N2-[2-fluoro-4-(3-oxomorpholine-4-yl)-phényl]-(2R,4R)-4-allyloxypyrrolidine-1,2-dicarboxamide,
N1-(4-éthynylphényl)-N2-[4-(3-oxomorpholine-4-yl)phényl]-(2R,4R)-4-propargyloxypyrrolidine-1,2-dicarboxamide,
N1-(4-éthynylphényl)-N2-[2-fluoro-4-(3-oxomorpholine-4-yl)-phényl]-(2R,4R)-4-propargyloxypyrrolidine-1,2-dicarboxamide,
N1-(4-éthynylphényl)-N2-[2-fluoro-4-(2-oxo-2H-pyrid ine-1-yl)-phényl]-(2R,4R)-4-méthoxypyrrolidine-1,2-dicarboxamide,
N1-(4-éthynylphényl)-N2-[4-(3-méthyl-2-oxo-2H-pyridine-1-yl)-phényl]-(2R,4R)-4-méthoxypyrrolidine-1,2-dicarboxamide,
N1-(4-éthynylphényl)-N2-[2-fluoro-4-(3-oxomorpholine-4-yl)-phényl]-(2R,4S)-4-propargyloxypyrrolidine-1,2-dicarboxamide,
N1-(4-éthynylphényl)-N2-[4-(3-oxomorpholine-4-yl)phényl]-(2R,4R)-4-(2,3-dihydroxypropoxy)pyrrolidine-1,2-dicarboxamide,
N1-(4-éthynylphényl)-N2-[4-(5-méthyl-2-oxo-2H-pyridine-1-yl)-phényl]-(2R,4R)-4-méthoxypyrrolidine-1,2-dicarboxamide,
N1-(4-éthynylphényl)-N2-[4-(2-méthoxycarbonyl-4-hydroxypyrrolidine-1-yl)phényl]-(2R,4R)-4-méthoxypyrrolidine-1,2-dicarboxamide,
N1-(4-éthynylphényl)-N2-[2-fluoro-4-(3-méthyl-2-oxo-2*H*-pyridine-1-yl)phényl]-(2S,4R)-4-méthoxypyrrolidine-1,2-dicarboxamide,
N1-(4-éthynylphényl)-N2-[2-fluoro-4-(3-méthyl-2-oxo-2*H*-pyridine-1-yl)phényl]-(2R,4R)-4-méthoxypyrrolidine-1,2-dicarboxamide,
N1-(4-éthynylphényl)-N2-[4-(3-oxomorpholine-4-yl)phényl]-(2R,4R)-4-(méthoxyéthoxy)pyrrolidine-1,2-dicarboxamide,
N1-(4-éthynylphényl)-N2-[4-(3-oxomorpholine-4-yl)phényl]-(2R,4R)-4-(méthoxycarbonylméthoxy)pyrrolidine-1,2-dicarboxamide,
N1-(4-éthynylphényl)-N2-[4-(3-oxomorpholine-4-yl)phényl]-(2R,4R)-4-(carboxyméthoxy)pyrrolidine-1,2-dicarboxamide,
N1-(4-éthynylphényl)-N2-[4-(6-méthyl-3-oxo-2*H*-pyridazine-2-yl)-phényl]-(2R,4R)-4-méthoxypyrrolidine-1,2-dicarboxamide,
N1-(4-éthynylphényl)-N2-[2-méthyl-4-(3-oxomorpholine-4-yl)-phényl]-(2R,4R)-4-(méthoxyéthoxy)pyrrolidine-1,2-dicarboxamide,
N1-(4-éthynylphényl)-N2-[2-fluoro-4-(3-oxomorpholine-4-yl)-phényl]-(2R,4R)-4-(méthoxyéthoxy)pyrrolidine-1,2-dicarboxamide.

13. Procédé pour la préparation de composés de la formule 1 conformément aux revendications 1 à 7, **caractérisé en ce que**
(a) un composé de la formule II 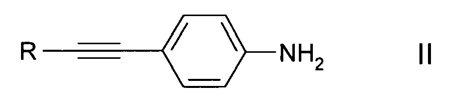 dans laquelle R présente la signification indiquée selon la revendication 1,
est amené à réagir avec un dérivé de chloroformate pour obtenir un intermédiaire dérivé de carbamate,
qui est ensuite amené à réagir avec un composé de la formule III 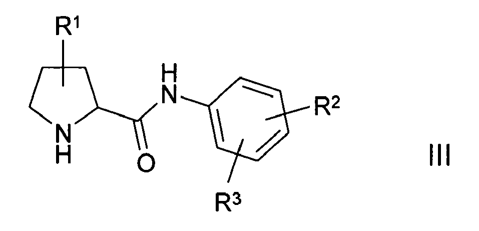 dans laquelle
R¹, R² et R³ présentent la signification indiquée selon la revendication 1,
ou
(b) un composé de la formule III
est amené à réagir avec un composé de la formule IV 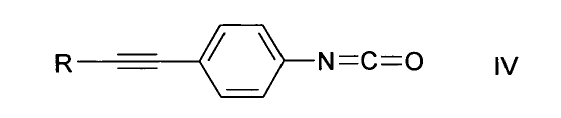 dans laquelle
R présente la signification indiquée selon la revendication 1,
ou
(c) un composé de la formule V 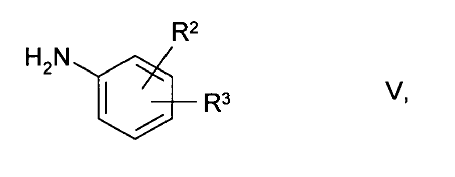 dans laquelle R² et R³ présentent la signification indiquée selon la revendication 1,
est amené à réagir avec un composé de la formule VI 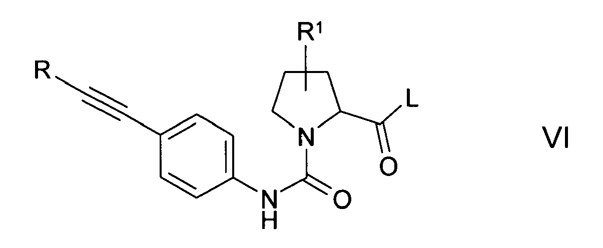
dans laquelle
L représente Cl, Br, I ou un groupe OH modifié fonctionnellement réactivement ou libre, et
R et R¹ présentent les significations indiquées selon la revendication 1,
et/ou une base ou un acide de la formule I est converti selon l'un de ses sels.

14. Composés de la formule I selon une ou plusieurs des revendications 1 à 12 en tant qu'inhibiteurs de facteur de coagulation Xa.

15. Composés de la formule I selon une ou plusieurs des revendications 1 à 12 en tant qu'inhibiteurs de facteur de coagulation VIIa.

16. Médicaments comprenant au moins un composé de la formule I selon une ou plusieurs des revendications 1 à 12 et/ou leurs sels, solvates et stéréo-isomères pharmaceutiquement utilisables, y compris des mélanges afférents selon tous rapports, et en option, des excipients et/ou des adjuvants.

17. Médicaments comprenant au moins un composé de la formule I selon une ou plusieurs des revendications 1 à 12 et/ou leurs sels, solvates et stéréo-isomères pharmaceutiquement utilisables, incluant des mélanges afférents selon tous rapports, et au moins un autre ingrédient actif de médicament.

18. Utilisation de composés selon une ou plusieurs des revendications 1 à 12 et/ou de leurs sels et solvates physiologiquement acceptables pour la préparation d'un médicament pour le traitement des thromboses, de l'infarctus du myocarde, de l'artériosclérose, des inflammations, de l'apoplexie, de l'angine de poitrine, de la resténose après angioplastie, de la claudication intermittente, de la migraine, du tintement, des tumeurs, des maladies tumorales et/ou des métastases tumorales.

19. Ensemble (kit) constitué par des packs séparés de
(a) une quantité efficace d'un composé de la formule I selon une ou plusieurs des revendications 1 à 12 et /ou de leurs sels, solvates et stéréo-isomères pharmaceutiquement utilisables, y compris des mélanges afférents selon tous rapports,
et
(b) une quantité efficace d'un autre ingrédient actif de médicament.

20. Utilisation de composés de la formule I selon une ou plusieurs des revendications 1 à 12 et/ou de leurs sels, solvates et stéréo-isomères pharmaceutiquement utilisables, y compris des mélanges afférents selon tous rapports, pour la préparation d'un médicament pour le traitement des thromboses, de l'infarctus du myocarde, de l'artériosclérose, des inflammations, de l'apoplexie, de l'angine de poitrine, de la resténose après angioplastie, de la claudication intermittente, de la migraine, du tintement, des tumeurs, des maladies tumorales et/ou des métastases tumorales,
en combinaison avec au moins un autre ingrédient actif de médicament.
